# EUROPÄISCHE PATENTSCHRIFT

(11) **EP 3 108 762 B1**
(45) Veröffentlichungstag und Bekanntmachung des Hinweises auf die Patenterteilung: **08.01.2020**
(21) Anmeldenummer: 16175350.4
(22) Anmeldetag: 20.06.2016
(51) Int. Cl.: A61F 2/44, A61F 2/30, A44B 18/00

(54) **BAUSATZ ZUM AUFBAU EINES KÄFIGS FÜR DIE SPONDYLODESE UND VERFAHREN HIERZU**
KIT FOR BUILDING A CAGE FOR SPONDYLODESIS AND METHOD THEREFOR
ENSEMBLE DE CONSTRUCTION POUR LA CONSTRUCTION D'UNE CAGE POUR LA SPONDYLODÈSE ET PROCÉDÉ POUR CELA

(30) Priorität: 25.06.2015 DE 102015110202; 09.06.2016 CA 2932759
(43) Veröffentlichungstag der Anmeldung: 28.12.2016
(73) Patentinhaber: Heraeus Medical GmbH, 61273 Wehrheim (DE)
(72) Erfinder: Vogt, Sebastian Dr., 99092 Erfurt (DE); Kluge, Thomas Dr., 56179 Vallendar (DE)
(74) Vertreter: Schultheiss & Sterzel Patentanwälte PartG mbB

(56) Entgegenhaltungen:
- US-A1- 2004 249 471
- US-A1- 2006 015 184
- US-A1- 2007 100 452
- US-A1- 2010 286 778
- US-A1- 2014 207 245

## Beschreibung

Die Erfindung betrifft einen modularen Bausatz zur Herstellung eines Käfigs für die Spondylodese. Die Erfindung betrifft auch ein Verfahren zur Herstellung eines solchen Käfigs und einen Käfig zur Spondylodese.

Gegenstand der Erfindung ist somit auch ein Käfig (häufig im internationalen Sprachgebrauch auch als "Cage" bezeichnet) zur Spondylodese von Wirbelkörpern mit einstellbarer Höhe und ein Verfahren zur Herstellung von Käfigen ("Cages") mit einstellbarer Höhe.

Die Spondylodese (Wirbelkörperverblockung) wird zur Versteifung von zwei oder mehreren Wirbelkörpern der Hals-, Brust- und Lendenwirbelsäule eingesetzt. Die Spondylodese (Wirbelkörperverblockung) kommt bei instabilen Brüchen von Wirbelkörpern, bei kongenitaler (angeborener) Skoliose und bei Wirbelgleiten zur Anwendung. Dabei werden zwei oder mehrere übereinander liegende Wirbelkörper mit Hilfe von Schrauben, Platten und Stangen miteinander verbunden. In den Zwischenwirbelraum werden Käfige (Cages) eingesetzt, welche die übereinander liegenden Wirbelkörper beabstanden, so dass eine Kompression des Rückenmarks und der davon ausgehenden Nerven vermieden wird. Weiterhin dienen die Käfige der Kraftübertragung zwischen den Wirbelkörpern. Die Käfige enthalten üblicherweise einen offenen axialen Hohlraum. Diese Hohlraum kann zum Beispiel mit Knochenersatzmaterialien, wie Calciumphosphaten, oder auch mit autologer Spongiosa gefüllt werden (Aaron R Cutler A. R., et. Al.: Comparison of polyetheretherketone cages with femoral cortical bone allograft as a single-piece interbody spacer in transforaminal lumbar interbody fusion. J. of Neurosurgery. Spine 5, Nr. 6 (2006): 534-539.). In diesen Hohlraum kann Knochengewebe zwischen den Wirbelkörpern einwachsen, so dass die Wirbelkörper über den neugebildeten Knochen verbunden sind. Für den klinischen Erfolg der Käfige ist es wesentlich, dass der physiologisch korrekte Abstand zwischen den Wirbelkörpern durch die Höhe der eingesetzten Käfige eingehalten wird. Das bedeutet, dass es unbedingt vermieden werden muss, dass die Käfige zu hoch oder zu niedrig sind. Üblich ist es daher, dass Käfige in verschieden Höhen von den Herstellern angeboten werden. Alternativ dazu sind Käfige bekannt, bei denen die Höhe des Käfigs individuell eingestellt werden kann. Exemplarisch sind dafür die Patentanmeldungen US 2014/358 235 A1, CA 2 846 793 A1, CA 2 845 613 A1 und US 2014/207 238 A1. Die US 2006/015 184 A1 offenbart Platten für die Spondylodese, die einzeln oder gruppenweise über Vorsprünge und Vertiefungen miteinander verbunden werden können, um eine Wirbelverblockung mit variabler Höhe zu erhalten. Diese Käfige sind mechanisch anspruchsvoll und relativ kompliziert aufgebaut. Zudem sind sie aufgrund des komplexen Aufbaus relativ kostspielig und nicht einfach in der Handhabung.

Die Aufgabe der Erfindung besteht also darin, die Nachteile des Stands der Technik zu überwinden. Insbesondere soll ein mechanisch stabiler Käfig für die Spondylodese entwickelt werden, dessen Höhe ohne Verwendung von technischen Hilfsmitteln in einfachster Weise eingestellt werden kann. Der zu entwickelnde Käfig soll keine komplexen Hebel- und/oder Schraubsysteme enthalten. Des Weiteren soll der Käfig möglichst porös sein, um ein eine knöcherne Durchbauung des Käfigs zu gewährleisten. Weiterhin muss der Käfig biokompatibel sein. Der Käfig soll auch einen formstabilen porösen Körper bilden, eine offene Porosität besitzen sowie mechanisch stabil sein. Die Porosität und die Größe der Poren sollen dabei dazu ausreichen und dazu geeignet sein, dass menschlicher Knochen eines Patienten, der mit dem Käfig behandelt wird, in die Poren des Käfigs einwachsen kann.

Die Aufgaben der Erfindung werden gelöst durch einen modularen Bausatz zur Herstellung eines Käfigs für die Spondylodese, der Bausatz aufweisend zumindest zwei Platten, wobei die Platten aus einem biokompatiblen Material bestehen und jeweils ein Flächengebilde aufweisen und eine Mehrzahl von sich aus dem Flächengebilde der Platten heraus erstreckende Stifte aufweisen, wobei die Stifte jeweils mindestens ein Rastelement aufweisen, wobei die Stifte elastisch verformbar sind und auf dem Flächengebilde derart dicht nebeneinander angeordnet sind, so dass durch Aufeinanderpressen von mit Stiften besetzten Flächengebilden mehrerer Platten die Rastelemente verschiedener Platten miteinander rasten, wobei zumindest zwei der zumindest zwei Platten eine Ausnehmung mit einem Durchmesser von wenigstens 5 mm aufweisen.

Bevorzugt kann vorgesehen sein, dass die zumindest zwei Platten aus einem biokompatiblen Kunststoff, einem biokompatiblen Metall und/oder einer biokompatiblen Metalllegierung bestehen. Biokompatible Metalle und biokompatible Metalllegierungen werden erfindungsgemäß bevorzugt, um daraus die Platten des Bausatzes herzustellen.

Bevorzugt weisen alle der zumindest zwei Platten eine Ausnehmung mit einem Durchmesser von wenigstens 5 mm auf.

Bei einem Rasten greifen die Rastelemente der Stifte der Platten derart in Rastelemente von Stiften benachbarter Platten, dass die Platten nicht mehr voneinander getrennt werden können aber auch nicht mehr durch weiteres Zusammendrücken der Platten aufeinander zu bewegt werden können. Die Rastelemente können durch Haken, Nuten, Hinterschnitte, Rastungen und/oder Gegenrastungen gebildet werden.

Unter dem Begriff "flächig" werden plane Körper und von planen Körpern abgeleitete Körper verstanden, die aus jeweils einem geschlossenen oder aus einem perforierten plattenartigen Grundkörper gebildet sind. Perforierte Flächengebilde sind dabei für die mit den Wirbelkörpern zu verbindenden Platten bevorzugt, weil durch diese Perforationen beziehungsweise die durch die Perforation gebildeten Poren Knochengewebe in die Platten einwachsen kann. Ganz besonders vorteilhaft und erfindungsgemäße bevorzugt ist es, wenn neben jedem Stift eine Perforation beziehungsweise eine Pore in der Platte angeordnet ist. Dann bilden sich nach der Rastung von mehreren Platten zwei poröse Grundflächen zur Verbindung mit den Wirbelkörpern, die bei geeigneter Materialwahl, wie zum Beispiel Tantal, osteokonduktiv sind.

Bei Druckeinwirkung auf die miteinander in Berührung stehenden Platten des Bausatzes bilden diese erfindungsgemäß einen mechanisch stabilen Verbund aus.

Bei erfindungsgemäßen Bausätzen kann vorgesehen sein, dass die miteinander gerasteten Platten einen Käfig aus miteinander gerasteten Platten mit zumindest einem offenen axialen Hohlraum bilden, wobei der Hohlraum einen Durchmesser von wenigstens 5 mm hat, wobei bevorzugt die miteinander gerasteten Platten einen Käfig aus miteinander gerasteten Platten mit zwei offenen axialen Hohlräumen bilden, wobei die beiden Hohlräume einen Durchmesser von jeweils wenigstens 5 mm hat.

Hierdurch wird von dem zumindest einen offenen axialen Hohlraum das Innere des Käfigs gebildet. Durch den zumindest einen offenen axialen Hohlraum kann der Knochen durchwachsen, so dass die beiden Wirbelkörper miteinander verwachsen können.

Des Weiteren kann vorgesehen sein, dass jede der zumindest zwei Platten zumindest eine Ausnehmung mit einem Durchmesser von wenigstens 5 mm aufweist, wobei bevorzugt mit den Ausnehmungen der zumindest zwei Platten der zumindest eine offene axiale Hohlraum des aus den Platten gefertigten Käfigs herzustellen ist.

Hierdurch können durch aufeinanderlegen und miteinander rasten der zumindest zwei Platten die Ausnehmungen übereinander liegend zueinander angeordnet werden, so dass die übereinander liegenden Ausnehmungen den zumindest einen offenen axialen Hohlraum bilden in die der Knochen von den beiden angrenzenden Wirbelkörpern einwachsen kann.

Mit der Erfindung wird auch vorgeschlagen, dass die Wandung des zumindest einen offenen axialen Hohlraums eines aus den zumindest zwei gerasteten Platten gebildeten Käfigs und/oder die Begrenzungen der Ausnehmungen der zumindest zwei Platten mit autologem Knochenmaterial gefüllt sind.

Hierdurch wird das autologe Knochenwachstum gefördert, so dass die Ausnehmungen beziehungsweise der zumindest eine offene axiale Hohlraum leichter und schneller vom Knochen des Patienten durchwachsen werden können.

Mit einer Weiterbildung wird vorgeschlagen, dass die miteinander gerasteten Platten einen porösen Käfig aus miteinander gerasteten Platten bilden, bevorzugt einen offenporigen Käfig aus miteinander gerasteten Platten bilden.

Hierdurch kann der Knochen zumindest bereichsweise in die Poren des aus den Platten geformten Käfigs wachsen. Des Weiteren wird mit der Erfindung vorgeschlagen, dass die Poren des aus mehreren Platten gebildeten offenporigen Käfigs interkonnektierend und osteokonduktiv sind, wobei vorzugsweise die Poren einen freien Querschnitt zwischen 0,1 mm und 1 mm aufweisen, besonders bevorzugt zwischen 0,25 mm und 0,9 mm. Hiermit wird sichergestellt, dass der Knochen gut mit den Poren des aus dem Bausatz gebildeten Käfigs verwachsen kann. Die nach außen weisenden Seitenwände des Käfigs können erfindungsgemäß durch eine geschlossene Wandung abgeschlossen sein. Hierzu kann an den zumindest zwei Platten ein umlaufender Rand vorgesehen sein, der bei Rastung mit einer benachbarten Platte mit dem Rand der benachbarten Platte formschlüssig abschließt und so die geschlossene Wandung bildet.

Bei bevorzugten Bausätzen kann erfindungsgemäß vorgesehen sein, dass der modulare Bausatz zur Herstellung eines Käfigs für die Spondylodese eine einstellbare Höhe aufweist und hierzu vorzugsweise zumindest drei Platten aufweist, damit durch die optionale Verwendung einer inneren Platte oder von mehreren inneren Platten verschiedene Höhen einstellbar sind.

Hierdurch wird ein besonders leicht aus dem Bausatz mit verschiedenen Höhen aufzubauender Käfig ermöglicht. Der Bausatz ist dadurch für unterschiedliche Patientenanforderungen variabel einsetzbar und an die anatomische Verhältnisse anzupassen.

Bei einer Weiterbildung kann auch vorgesehen sein, dass die zumindest zwei Platten einen abschließenden umlaufenden Rand aufweisen, so dass die miteinander gerasteten Platten einen Käfig mit einer nach außen abgeschlossenen Wandung bilden, wobei vorzugsweise die Ränder ineinander verzahnt sind.

Hierdurch kann verhindert werden, dass Material aus dem Inneren des hergestellten Käfigs nach außen dringen kann.

Ferner kann im Rahmen der vorliegenden Erfindung vorgesehen sein, dass zumindest eine Gruppe der zumindest zwei Platten, bevorzugt zumindest eine Gruppe der zumindest vier Platten, bezüglich der Form der Flächengebilde gleichförmig oder im Wesentlichen gleichförmig sind, so dass sich diese in der Richtung senkrecht zu den Flächengebilden formschlüssig aufeinander stapeln und/oder rasten lassen.

Hierdurch kann mit dem Bausatz ein bezüglich der Höhe variabler aber im Umfang eindeutig geformter Käfig aus dem Bausatz hergestellt werden.

Dabei kann vorgesehen sein, dass zumindest zwei Gruppen der zumindest zwei Platten, bevorzugt zumindest zwei Gruppen der zumindest vier Platten, bezüglich der Form der Flächengebilde gleichförmig oder im Wesentlichen gleichförmig sind, so dass sich diese in der Richtung senkrecht zu den Flächengebilden formschlüssig aufeinander stapeln und/oder rasten lassen, wobei die zumindest zwei Gruppen unterschiedliche Geometrien bezüglich der Ebenen der Flächengebilde aufweisen.

Hierdurch kann mit dem Bausatz eine Mehrzahl verschiedener Käfige für unterschiedliche anatomische Verhältnisse aufgebaut werden.

Ferner kann vorgesehen sein, dass zumindest zwei äußeren Platten der zumindest zwei Platten, die zum direkten Verbinden mit den Wirbelkörpern vorgesehen sind, durch Poren in den Flächengebilden osteokonduktiv sind und/oder die Flächengebilde eine Befestigungs-Oberfläche ohne Stifte aufweisen, die zum Anlegen an die Wirbelkörper vorgesehen ist, wobei bevorzugt die Befestigungs-Oberfläche Spitzen oder Noppen zum Verbinden der Platten mit dem Knochen der Wirbelkörper aufweist.

Die Spitzen oder Noppen können in den Knochen der Wirbelkörper eingedrückt werden. Alternativ oder zusätzlich können in dem Flächengebilde der zumindest zwei äußeren Platten Ösen oder Bohrungen vorgesehen sein, durch die die zumindest zwei äußeren Platten auf einen Wirbelkörper geschraubt oder anders (beispielsweise mit Nägeln befestigt werden können. Auch wenn beide Seiten der zumindest zwei Platten Stifte aufweisen, können scharfe Spitzen zur Befestigung an der Knochenoberfläche vorgesehen sein, die Spitzen sollten hierzu die Stifte mit den Rastelementen überragen. Bei Ausführungsformen mit scharfen Spitzen kann eine Platte durch Einschlagen der scharfen Spitzen in das Knochengewebe der Wirbelkörper verankert werden. Auf diesem aufbauend können dann weitere Platten des Bausatzes aufgebracht und gerastet werden.

Derartige äußere Platten können zur unmittelbaren flächigen Befestigung am Knochen verwendet werden. Mit den Ösen oder Bohrungen ist es möglich, die Platten am Knochengewebe anzuschrauben und auf diese Platte beliebig viele weitere Platten des Bausatzes aufzubringen und anschließend zu rasten. Dadurch lassen sich dreidimensionale Käfige mit variabler Höhe lastragend aufbauen.

Erfindungsgemäß bevorzugt sind die Poren abgerundet, insbesondere weisen sie keine scharfkantigen Konturen auf. Besonders bevorzugt weisen die Poren in dem Flächengebilde der Platte einen freien Querschnitt zwischen 0,25 mm und 1 mm auf, besonders bevorzugt zwischen 0,3 mm und 0,9 mm.

Durch die Poren kann der aus den Platten des Bausatzes gefertigte Käfig besonders stabil mit den angrenzenden Wirbelkörpern befestigt werden.

Gemäß einer bevorzugten Ausführung des erfindungsgemäßen Bausatzes kann vorgesehen sein, dass zumindest drei Platten vorgesehen sind und dabei zumindest eine innere Platten und zumindest eine äußere Platten, wobei immer eine innere Platte und dazu jeweils wenigstens eine äußere Platte eine Flächengruppe bilden, wobei jede äußere Platte eine Ausnehmung aufweist, so dass diese äußere Platte die innere Platte der gleichen Flächengruppe oder eine andere äußere Platte der gleichen Flächengruppe in der Ebene der Flächengebilde formschlüssig umschließt.

Hierdurch können mit dem Bausatz Käfige unterschiedlicher Querschnitte gefertigt werden.

Des Weiteren kann vorgesehen sein, dass die Flächengebilde zumindest einer der Platten, insbesondere zumindest einer inneren Platte, einen Gradienten in der Dicke aufweist, wobei vorzugsweise der Bereich mit der größten Dicke um maximal 100% dicker ist als der Bereich mit der geringsten Dicke.

Durch einen derartigen Bausatz können auch gekrümmte oder geneigte Käfige hergestellt werden, mit denen die Stellung der Wirbelkörper zueinander angepasst beziehungsweise berücksichtigt werden kann.

Es wird ferner vorgeschlagen, dass an einem Flächengebilde wenigstens einer Platte, bevorzugt an den Flächengebilden der äußeren Platten, zumindest zwei Positionierhilfen, insbesondere Positionierstifte, vorgesehen sind, wobei mit den Positionierhilfen die Orientierung der zusammenzufügenden Platten zueinander vorgegeben ist.

Hiermit wird sichergestellt, dass die Platten nur in der gewünschten Orientierung zusammensetzbar sind. Dadurch wird die Möglichkeit für Fehlanwendungen reduziert.

Bevorzugt kann vorgesehen sein, dass die Rastelemente Pilze, Haken, Hinterschnitte, Rastungen und/oder Gegenrastungen sind.

Diese Rastelemente sind besonders gut zur gegenseitigen Rastung geeignet. Textile Verbindungen, wie beispielsweise Klettverbindungen mit leicht verformbaren Fasern, sind dagegen erfindungsgemäß ungeeignet, da damit keine formstabilen und druckstabilen Käfige für die Spondylodese aufbaubar sind.

Mit einer Weiterbildung wird vorgeschlagen, dass der Abstand zwischen den Rastelementen und dem Flächengebilde der zumindest zwei Platten zwischen 0,3 mm und 2 mm beträgt, bevorzugt zwischen 0,5 mm und 1 mm beträgt.

Hierdurch wird eine ausreichend stabile Rastung zwischen den Rastelementen erreicht und gleichzeitig ein ausreichendes Verbiegen der Stifte zur Verbindung der Rastelemente ermöglicht.

Bei einer Weiterentwicklung kann vorgesehen sein, dass zumindest eines der zumindest einen Rastelemente pro Stift stumpf-kegelförmig ausgebildet ist, wobei die Längsachsen der Stifte die Längsachsen der Kegel bilden und wobei der Mantel der Kegel nach der zu dem Flächengebilde der zumindest einen Platte abgewandten Außenseite gerichtet ist.

Hierdurch wird eine besonders stabile Verbindbarkeit der zumindest zwei Platten über die als Kegelstümpfe geformten Rastelemente erreicht. Zudem wird durch diese Formgebung nach der Implantation des Käfigs verhindert, dass umgebendes Weich- und Knochengewebe verletzt wird.

Des Weiteren kann vorgesehen sein, dass zumindest eines der zumindest einen Rastelemente pro Stift als Haken und/oder als Pilzkopf ausgebildet ist.

Die Haken und/oder die Pilzköpfe sorgen für eine stabile und unlösbare Verbindung der Platten untereinander. Wenn die Rastelemente als Pilzköpfe geformt sind, können diese zum Beispiel einen in Richtung des Flächengebildes ausgebildeten Kragen am Pilzkopfrand besitzen, so dass in diesem so erzeugten Hinterschnitt hakenförmige Rastelemente von anderen Platten eingreifen können, wodurch eine irreversible, nicht lösbare Verhakung oder Rastung zwischen den Platten entsteht. Es ist auch möglich und erfindungsgemäß bevorzugt, dass zumindest eine Platte verschiedene Rastelemente enthält oder verschiedene Stifte mit unterschiedlichen Rastelementen aufweist. So kann eine Platte zum Beispiel Haken und Pilzköpfe als Rastelemente gleichzeitig besitzen sowohl am gleichen Stift als auch an unterschiedlichen Stiften.

In einer bevorzugten Ausführungsform sind die Rastelemente als Pilzköpfe ausgebildet. In einer besonders bevorzugten Ausführungsform sind die Pilzköpfe so gestaltet, dass die Pilzköpfe an der, der Oberfläche des jeweiligen Flächengebildes zugewandten Seite, einen kegelförmigen Hinterschnitt aufweisen. Dadurch können hakenförmige Rastelemente mit diesen Pilzköpfen irreversibel und unlösbar verhakt werden. Bei geeigneter zueinander passender Form der Hinterschnitte zu den Pilzköpfen kann ein weiteres Vortreiben der Pilzköpfe verhindert werden, so dass die Pilzköpfe mit den Hinterschnitten rasten.

Gemäß einer bevorzugten Weiterentwicklung kann vorgesehen sein, dass die Stifte zwischen dem Flächengebilde der zumindest zwei Platten und zumindest einem der zumindest einen Rastelemente eine umlaufende Nut als Gegenrastmittel enthalten, in welche Rastelemente anderer Platten einrasten können, bevorzugt derart einrasten, dass keine weitere Bewegung der Rastelemente entlang der Stifte möglich ist.

Auch hierdurch wird eine besonders stabile Verbindung von Platten untereinander ermöglicht.

Besonders vorteilhafte Bausätze können vorsehen, dass die zumindest zwei Platten aus biokompatiblem Kunststoff, Edelstahl, Titan, einer Titanlegierung, Tantal, einer Tantallegierung oder aus Kompositen dieser Materialien gebildet sind.

Diese Materialien sind für medizinische Zwecke besonders gut einsetzbar und mit diesen Materialien lassen sich auch die geeigneten elastischen Eigenschaften für die Stifte einstellen. Aus Metall oder Metalllegierungen bestehende Platten können erfindungsgemäß bevorzugt durch selektives Lasersintern oder auch durch Schmelzen mit Elektronenstrahlen hergestellt werden, bevorzugt mit einem 3D-Druckverfahren.

Der biokompatible Kunststoff kann biodegradierbar sein. Dazu können Polylactide, Polylglycolide, Polycaprolactone und Polyester verwendet werden, die aus unterschiedlichen α-Hydroxycarbonsäuren gebildet sind. Als nicht biodegradierbare Kunststoffe kommen Polyamide, Polyimide, Polyetherketon und Polysulfon in Betracht. Platten aus diesen nicht biodegradierbaren und biodegradierbaren Kunststoffen können durch selektives Lasersintern hergestellt werden.

Gemäß einer bevorzugten Ausführung der vorliegenden Erfindung wird vorgeschlagen, dass benachbarte Stifte, die auf der gleichen Seite einer ersten Platte der zumindest zwei Platten angeordnet sind, einen derartigen Abstand zueinander aufweisen, dass nach einer elastischer Deformation aufgrund einer Rastung mit einem Rastelement einer zweiten Platte der zumindest zwei Platten, die Stifte der ersten Platte mindestens zwei Rastungen mit zumindest zwei weiteren Rastelementen der zweiten Platte ermöglichen, bevorzugt mindestens drei Rastungen mit drei weiteren Rastelementen der zweiten Platte ermöglichen.

Durch eine mehrfache Rastung der Platten kann ein besonders stabiler Käfig aus den Platten des Bausatzes geformt werden.

Es kann auch vorgesehen sein, dass die zumindest zwei Platten mit anorganischem oder organischem partikulärem Knochenersatzmaterial und/oder autologer oder auch allogener Spongiosa gefüllt sind.

Hiermit kann die Knochenheilung und die Verbindung des Käfigs mit dem Knochen der Wirbelkörper beschleunigt werden.

Ferner kann vorgesehen sein, dass die zumindest zwei Platten mit einem oder mehreren pharmazeutischen Wirkstoffen aus den Gruppen der Antibiotika, der Bisphosphonate, der Steroide, der nichtsteroidalen Entzündungshemmer, der Wachstumsfaktoren und der Cytostatika beschichtet sind.

Hiermit wird eine pharmakologische Wirkung des Käfigs erreicht, die zur Heilung des mit dem Käfig behandelten Patienten beiträgt. Aus der Gruppe der Antibiotika sind besonders Gentamicin, Tobramycin, Amikacin, Vancomycin, Teicoplanin, Clindamycin und Daptomycin bevorzugt.

Des Weiteren kann vorgesehen sein, dass die Stifte in Reihen von jeweils drei oder mehreren Stiften angeordnet sind und dass zwischen diesen jeweils drei oder mehrere Reihen ein Streifen von unbesetzter Oberfläche der Flächengebilde verbleibt oder dass eine gruppierte oder nestförmige Anordnung von Stiften mit Rastelementen vorgesehen ist.

Hierdurch wird Raum für die Verformung der Stifte mit den Rastelementen bei der Rastung gegeben.

Ferner kann vorgesehen sein, dass die Stifte der zumindest zwei Platten sich senkrecht oder mit einem Winkel zwischen 60° und 90°, bevorzugt mit einem Winkel zwischen 80° und 90°, aus den Flächengebilden der zumindest zwei Platten erstrecken.

Hierdurch wird erreicht, dass sich die Platten später besonders leicht miteinander verbinden lassen. Zudem wird so eine gleichmäßige Belastbarkeit des Käfigs erreicht.

Gemäß einer bevorzugten Ausführung der vorliegenden Erfindung kann vorgesehen sein, dass der Bausatz zumindest zwei äußere Platten zum Verbinden mit den Wirbelkörpern und zumindest eine innere Platte zum Einstellen der Höhe des zu erzeugenden Käfigs aufweist, wobei jede der zumindest einen inneren Platte auf beiden Seiten des Flächengebildes Stifte mit Rastelementen aufweist und bevorzugt die zumindest zwei äußeren Platten nur auf einer Seite der Flächengebilde Stifte mit Rastelementen aufweisen.

Hierdurch kann mit dem Bausatz besonders einfach durch Weglassen oder Einfügen vom inneren Platten die Höhe des zu erzeugenden Käfigs eingestellt werden.

Es wird auch vorgeschlagen, dass die Rastelemente an der Mantelfläche der Stifte ausgebildet sind.

Damit kann eine stabile Verbindung der Stifte und damit der Platten untereinander erreicht werden.

Bevorzugte erfindungsgemäße Bausätze können vorsehen, dass ineinander gepresste Platten irreversibel miteinander verhaken und/oder rasten.

Hierdurch wird sichergestellt, dass sich Platten des fertig geformten Käfigs nicht von dem Käfig lösen.

Mit einer Weiterbildung der vorliegenden Erfindung wird vorgeschlagen, dass die zumindest zwei Platten ohne die hervorragenden Stifte beziehungsweise die Flächengebilde der Platten eine Stärke von höchstens 2 mm aufweisen, bevorzugt einen Stärke zwischen 0,25 mm und 1,5 mm aufweisen, besonders bevorzugt einen Stärke zwischen 0,5 mm und 1,5 mm aufweisen.

Die Stärke der zumindest zwei Platten beziehungsweise der Flächengebilde kann auch als die Dicke der Platten beziehungsweise Flächengebilde bezeichnet werden und ist die Abmessung der Platte ohne die Stifte, die senkrecht zur dem Flächengebilde der Platte angeordnet ist. Damit wird erreicht, dass die Platten entweder ausreichend stark gebogen beziehungsweise verformt werden können, um an die Behandlungssituation angepasst zu werden, oder dass verschiedene Bauhöhen des Käfigs durch den Einsatz unterschiedlich dicker Platten mit nur wenigen Platten erreicht werden kann.

Es kann des Weiteren vorgesehen sein, dass die zumindest zwei Platten mit einem generativen 3D-Druckverfahren hergestellt werden.

Hierdurch lassen sich die Platten und damit der Käfig kostengünstig herstellen.

Es kann auch vorgesehen sein, dass zwei Rastelemente hintereinander auf der Mantelfläche der Stifte angeordnet sind, besonders bevorzugt drei Rastelemente hintereinander auf der Mantelfläche der Stifte angeordnet sind.

Hierdurch wird es möglich, Platten mit unterschiedlichen Abständen zueinander zu rasten. Dadurch wird eine höhere Flexibilität beim Formen des Käfigs erreicht. Es ist dabei allerdings schwer die notwendige Stabilität des Käfigs senkrecht zur Ebene der Flächengebilde zu erreichen.

Es kann auch vorgesehen sein, dass die zumindest eine Platte in Form einer Fläche mit abgerundeten Ecken, vorzugsweise in Nierenform ausgebildet ist.

Durch diese Formen sind die Platten beziehungsweise die daraus gefertigten Käfige besonders gut an die zu behandelnden Wirbelkörper angepasst.

Ferner kann vorgesehen sein, dass in dem Flächengebilde der zumindest zwei Platten durchgehende Poren enthalten sind, wobei die Tiefe der Poren senkrecht zu dem Flächengebilde der zumindest zwei Platten zumindest 0,25 mm beträgt, bevorzugt zumindest 0,4 mm beträgt.

Hierdurch kann sichergestellt werden, dass die Poren stabil und gleichförmig vom Knochen umschlossen werden. Die Platten bilden so ein spongiöses Geweben nach, dass einer normalen Knochenstruktur entspricht und mit dieser und damit mit den Wirbelkörpern gut verwachsen kann.

Es kann auch vorgesehen sein, dass die zumindest eine Platte in dem Flächengebilde plastisch oder elastisch verformbar ist.

Dadurch kann die zumindest eine Platte besonders leicht an verschiedene Behandlungssituationen angepasst werden.

Die der vorliegenden Erfindung zugrundeliegenden Aufgaben werden auch gelöst durch ein Verfahren zum Herstellen eines Käfigs für die Spondylodese mit einem solchen erfindungsgemäßen Bausatz, bei dem mehrere Platten gegeneinander gedrückt werden, wobei die Platten miteinander rasten und den Käfig ausbilden.

Bei diesem Verfahren kann auch vorgesehen sein, dass die Stifte mit den Rastelementen von den zumindest zwei Platten in Kontakt miteinander gebracht werden und dass anschließend durch Druck der Platten gegeneinander die Stifte mit den Rastelementen miteinander gerastet werden.

Bei Druckeinwirkung auf die miteinander in Berührung stehenden Platten des Bausatzes bilden diese erfindungsgemäß einen mechanisch stabilen Verbund aus.

Mit einer Weiterentwicklung wird vorgeschlagen, dass zwischen zwei äußere Platten je nach gewünschter Dicke des zu erzeugenden Käfigs keine, eine oder mehrere innere Platten eingesetzt werden, wobei die Platten durch aufeinanderdrücken miteinander gerastet und dadurch fest miteinander verbunden werden.

Die der Erfindung zugrundeliegenden Aufgaben werden ferner gelöst durch einen Käfig für die Spondylodese aufgebaut aus zumindest zwei Platten eines erfindungsgemäßen Bausatzes und/oder hergestellt mit einem erfindungsgemäßen Verfahren.

Schließlich werden die der vorliegenden Erfindung zugrundeliegenden Aufgaben auch gelöst durch die Verwendung eines solchen Käfigs für eine Spondylodese in der Unfallchirurgie, Orthopädie oder in der Veterinärmedizin.

Der Erfindung liegt die überraschende Erkenntnis zugrunde, dass mechanisch rastende Platten als Bauteile eines Bausatzes zur Herstellung eines Käfigs für die Spondylodese verwenden werden können. Dabei können die Platten schichtweise angeordnet werden, wobei der so geformte Käfig nach Ausbildung der gewünschten dreidimensionalen Struktur fest und nicht komprimierbar ist, ohne dass chemische Aushärtungsreaktionen, wie beispielsweise radikalische Polymerisationen, oder aufwendige eine aufwendige Mechanik zur stabilen Einstellung der Höhe notwendig sind. Die Platten sind bevorzugt in begrenztem Umfang flexibel und können so in eine passende Form gebracht werden und durch Druck miteinander rasten und dabei miteinander verbunden werden. Wenn die geformten Platten miteinander rasten stabilisieren sich die Platten untereinander, so dass der entstehende dreidimensionale Käfig fest und formstabil ist. Indem die Platten durch Aneinanderdrücken aus verschiedenen Richtungen mit ausreichender Kraft miteinander verbunden werden, kann sichergestellt werden, dass derart viele Verhakungen und Rastungen erfolgen, dass der erzeugte Käfig formstabil und mechanisch belastbar ist. Durch eine geeignete Form und Größe der Platten wird so ein Käfig gebildet, der für die medizinische Anwendung mechanisch stabil genug ist und zur Form der Behandlungssituation passend gestaltet werden kann. Der Knochen kann bevorzugt vorgesehene Poren des durch Druck verbundenen Käfigs einwachsen und sich so mit dem Käfig dauerhaft verbinden. Durch zumindest einen offenen axialen Hohlraum im Käfig kann der Knochen von den Wirbelkörpern aus zusammenwachsen und das Gelenk der Wirbelkörper versteifen.

Überraschend wurde gefunden, dass die Platten des erfindungsgemäßen Bausatzes an die Wirbelkörper schichtweise angelegt werden können und durch einfaches Komprimieren per Hand oder mit Hilfe eines Stößels zu einem homogenen Körper unter Rastung der einzelnen Schichten (beziehungsweise Platten) ausgehärtet werden können. Eine Aushärtung des erfindungsgemäßen Käfigs ist durch einfaches Komprimieren von über die Flächen aneinander liegender Platten möglich. Eine lasttragende Verbindung zweier Wirbelkörper ist mit dem erfindungsgemäßen flächigen Material (also den Platten des erfindungsgemäßen Bausatzes) möglich.

Mechanisch verhakende Systeme nach dem Bauprinzip von Kletten sind seit mehreren Jahrzehnten bekannt. Erstmalig wurde das Prinzip des Klettverschlusses von de Mestral in CH 295 638 A beschrieben. Dieses Prinzip wurde weiterentwickelt und findet bei unterschiedlichsten, reversibel schließenden Klettverschlüssen Verwendung. Beispielhaft für die Weiterentwicklung sind die Schriften DE 1 610 318 A1, DE 1 625 396 A1, US 5 077 870 A und US 4 290 174 A.

Eine interessante Weiterentwicklung erfolgte später, bei der reversibel verklettende Stahlbandsysteme für mechanisch hochbelastbare Anwendungen und für Anwendungen bei hohen Temperaturen entwickelt wurden (DE 10 2004 048 464 A1, DE 10 2006 015 100 A1, DE 10 2006 015 145 A1, DE 10 2006 015 148 A1).

Es wurde im Rahmen der vorliegenden Erfindung überraschend gefunden, dass solche Systeme beziehungsweise derartige Funktionsprinzipien für den Aufbau von Käfigen für die Spondylodese einsetzbar sind beziehungsweise auf Platten zur Herstellung von Käfigen für die Spondylodese übertragbar sind. Dabei kann es für die Käfige von Vorteil sein, dass derartige Verbindungen nicht dicht abschließen, sondern Zwischenräume als offenporige Struktur verbleiben. Die sich dadurch im Käfig bildenden interkonnektierenden Poren können mit dem Knochen verwachsen und damit eine stabile Verbindung zwischen dem Knochen und dem Käfig erzeugen. Hierzu muss sichergestellt werden, dass die Poren in den Platten einen ausreichenden freien Querschnitt aufweisen. Die Poren werden als osteokonduktiv bezeichnet, wenn der Knochen in die Poren einwachsen kann und sich somit mit dem aus den Platten gebildeten Käfig verbindet.

Die Erfindung liegt somit die Idee zu Grunde, dass ein Käfig aus mindestens einer distalen und einer proximalen Platte zusammengesetzt werden kann, wobei zwischen den Platten je nach gewünschter Höhe des Käfigs ein oder mehrere Zwischenplatten eingefügt werden können. Der Verbund der Platten wird durch "klettartige" Rastungen von Rastelementen erreicht, die an der Oberfläche der der Platten angeordnet sind. Die Platten können vom medizinischen Anwender durch einfaches axiales Zusammendrücken miteinander verbunden werden.

Ein beispielhaftes und erfindungsgemäß besonders bevorzugtes Ausführungsbeispiel der vorliegenden Erfindung ist ein Käfig ("Cage") mit einstellbarer Höhe, der zusammengesetzt ist aus mindestens zwei Platten, bei denen auf wenigstens einer Seite drei oder mehrere elastisch verformbare Stifte angeordnet sind, die an einem Stiftende jeweils mindestens ein Rastelement besitzen und wobei die jeweils mindestens drei oder mehreren Stifte der distalen Platte, der proximalen Platte und der Zwischenplatten so dicht beieinander stehen, dass bei Kontakt der Stifte der jeweils aneinander liegenden Platten diese unter Druckeinwirkung miteinander verhaken und einen in axialer Richtung druckfesten Cage ausbilden.

Der Aufbau der Platten wird derart gestaltet und dass durch Zusammenpressen der Platten sich kontaktierende Platten irreversibel miteinander rasten und einen Käfig aus miteinander gerasteten Platten bilden.

Ein erfindungsgemäßer Käfig ist beispielsweise zusammengesetzt aus
a) einer distale (äußere) Platte, auf deren proximalen Seite drei oder mehrere elastisch verformbare Stifte angeordnet sind, die an einem Stiftende jeweils mindestens ein Rastelement besitzen,
b) einer proximale (äußere) Platte, auf deren proximalen Seite drei oder mehrere elastisch verformbare Stifte angeordnet sind, die an einem Stiftende jeweils mindestens ein Rastelement besitzen,
c) ein oder mehrere Zwischenplatten (innere Platten), auf deren distalen und proximalen Seite jeweils drei oder mehrere elastisch verformbare Stifte angeordnet sind, die an einem Stiftende jeweils mindestens ein Rastelement besitzen, und
d) wobei die jeweils mindestens drei oder mehr Stifte der distalen Platte, der proximalen Platte und der Zwischenplatten so dicht beieinander stehen, dass bei Kontakt der Stifte der jeweils aneinander liegenden Platten diese unter Druckeinwirkung miteinander verhaken und rasten und einen in axialer Richtung druckfesten Käfig ausbilden.

Ganz besonders vorteilhaft ist es, wenn neben jedem Stift mit Rastelement eine Perforation in den Platten angeordnet ist, dann bildet sich nach der Verrastung von mehreren Platten ein offen poröser Körper aus, der bei geeigneter Materialwahl, wie zum Beispiel von Tantal, osteokonduktiv ist.

Erfindungsgemäß kann vorgesehen sein, dass die zumindest zwei Platten, insbesondere die proximale Platte, die distale Platte und die Zwischenplatten, ringförmig oder elliptisch ringförmig oder in Form von zwei aneinander liegenden Ringen ausgebildet sind. Das bedeutet, dass in den zumindest einen axialen Hohlraum der Käfige übliche Knochenersatzmaterialien, wie Tricalciumphosphat, oder auch autologes Knochenmaterial eingebracht werden kann. Dadurch wird eine knöcherne Durchbauung des Käfigs begünstigt.

Vorteilhaft ist es, wenn die zumindest zwei Platten, insbesondere die proximale Platte, die distale Platte und die Zwischenplatten, Perforationen enthalten, die einen Durchmesser im Bereich von 300 µm bis 3000 µm besitzen. Diese Perforationen erlauben ein Einwachsen von Knochengewebe.

Erfindungsgemäß ist weiterhin ein beispielhaftes Verfahren zur Herstellung eines Käfigs für die Spondylodese. Dieses Verfahren ist dadurch charakterisiert, dass zwischen zwei äußere Platten (der distalen Platte und der proximalen Platte) je nach gewünschter Höhe des Käfigs ein oder mehrere Zwischenplatten angeordnet werden und dass die Platten gegeneinander gedrückt werden, so dass die Rastelemente der Platten verhaken und durch Formschluss der Rastelemente einen Verbund der Platten ausgebildet wird. Bei dem Verfahren werden die Platten vorteilhaft so übereinander gelegt, dass die äußeren Ränder der Platten bündig übereinander angeordnet werden.

Im Folgenden werden weitere Ausführungsbeispiele der Erfindung anhand von zweiundzwanzig schematisch dargestellten Figuren erläutert, ohne jedoch dabei die Erfindung zu beschränken. Dabei zeigt:
Figur 1: eine schematische perspektivische Ansicht auf einen erfindungsgemäßen Käfig, der aus fünf Platten eines erfindungsgemäßen Bausatzes aufgebaut ist;
Figur 2: eine schematische perspektivische Ansicht auf zwei der Platten nach Figur 1, die nicht miteinander gerastet sind;
Figur 3: eine schematische Querschnittansicht durch die fünf gerasteten Platten des Käfigs nach Figur 1;
Figur 4: eine vergrößerte Darstellung einer schematischen Querschnittansicht durch die fünf gerasteten Platten des Käfigs nach Figur 1 ;
Figur 5: eine schematische Querschnittansicht durch die zwei nicht gerasteten Platten nach Figur 2;
Figur 6: eine schematische perspektivische Ansicht auf einen zweiten erfindungsgemäßen Käfig, der aus vier Platten eines zweiten erfindungsgemäßen Bausatzes aufgebaut ist;
Figur 7: eine schematische perspektivische Ansicht des Käfigs nach Figur 6, der zwischen zwei Wirbelkörpern eingesetzt ist;
Figur 8: eine schematische Querschnittansicht durch die vier gerasteten Platten des Käfigs nach Figur 6;
Figur 9: eine vergrößerte Darstellung einer schematischen Querschnittansicht durch zwei gerastete Platten des Bausatzes nach den Figuren 6 bis 8;
Figur 10: eine schematische perspektivische Ansicht auf eine der Platten nach einer der Figuren 6 bis 9;
Figur 11: eine Vergrößerung eines Teilbereichs A in Figur 10;
Figur 12: eine schematische perspektivische Ansicht auf eine der Platten nach einer der Figuren 6 bis 9;
Figur 13: eine schematische Seitenansicht auf fünf am Rand miteinander verzahnte Platten, die einen dritten erfindungsgemäßen Käfig bilden;
Figur 14: eine schematische perspektivische Detailansicht auf zwei miteinander verhakte Haken (links) zwei nicht miteinander verhakte Haken (rechts) als Teile von Platten eines vierten erfindungsgemäßen Bausatzes;
Figur 15: eine schematische perspektivische Ansicht auf Ausschnitte von zwei nicht miteinander verhakten Platten eines fünften erfindungsgemäßen Bausatzes;
Figur 16: drei schematische Ansichten der Ausschnitte von zwei miteinander verbundenen Platten (nach Figur 15) des fünften Bausatzes A) Aufsicht auf die Oberseite, B) Seitenansicht, C) Querschnittansicht entlang des Schnitts B-B nach Figur 16 A);
Figur 17: Figur 17 Oben: eine schematische Querschnittansicht durch eine äußere Platte eines sechsten erfindungsgemäßen Bausatzes und Figur 17 Unten: eine schematische Seitenansicht auf die äußere Platte des sechsten Bausatzes;
Figur 18: eine vergrößerte Darstellung einer schematischen Querschnittansicht durch zwei gerastete Platten des sechsten Bausatzes nach Figur 17;
Figur 19: Figur 19 Oben: eine schematische Querschnittansicht durch eine äußere Platte eines siebten erfindungsgemäßen Bausatzes und Figur 19 Unten: eine schematische Seitenansicht auf die äußere Platte des siebten Bausatzes;
Figur 20: eine vergrößerte Darstellung einer schematischen Querschnittansicht durch zwei gerastete Platten des siebten Bausatzes nach Figur 19;
Figur 21: Figur 21 Oben: eine schematische Querschnittansicht durch eine äußere Platte eines achten erfindungsgemäßen Bausatzes und Figur 21 Unten: eine schematische Seitenansicht auf die äußere Platte des achten Bausatzes; und
Figur 22: eine vergrößerte Darstellung einer schematischen Querschnittansicht durch zwei gerastete Platten des achten Bausatzes nach Figur 21.

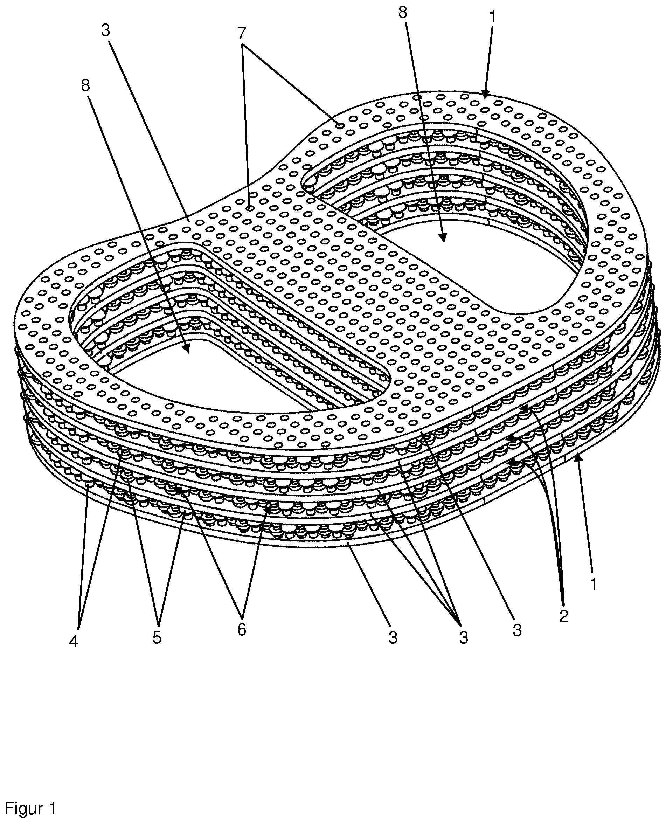

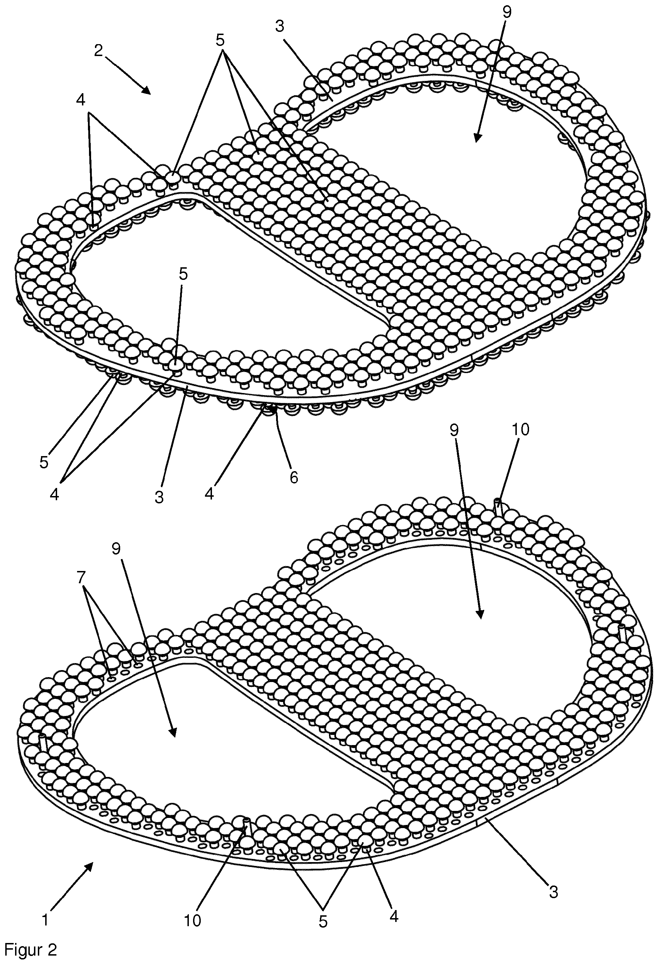

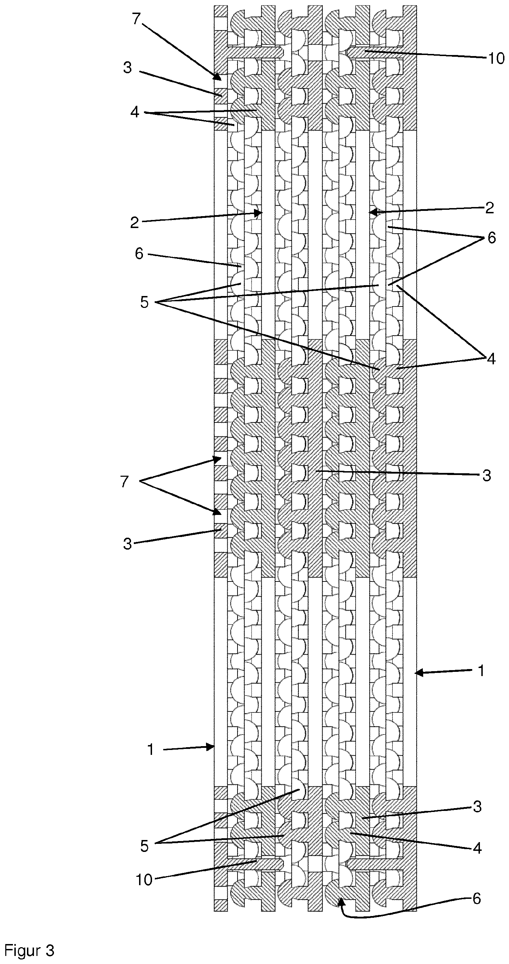

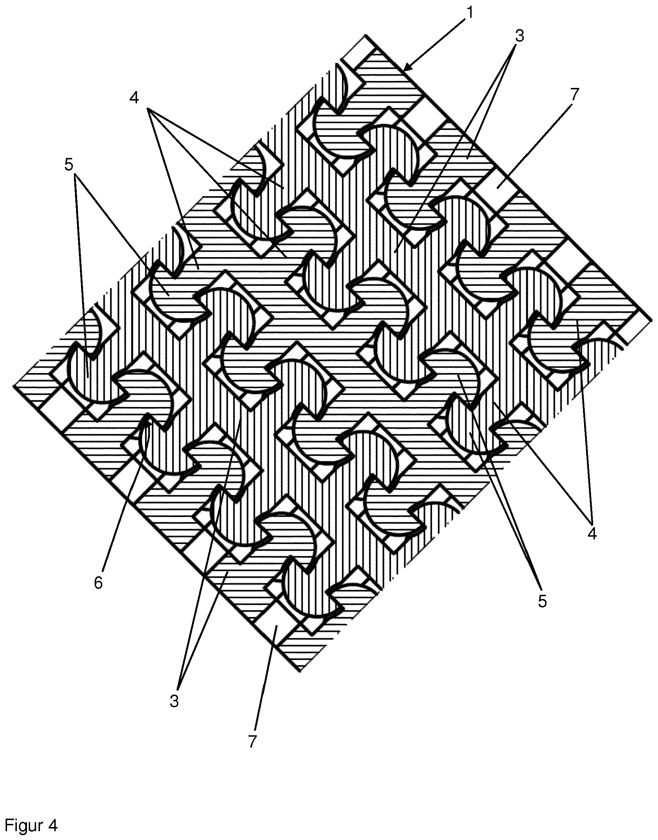

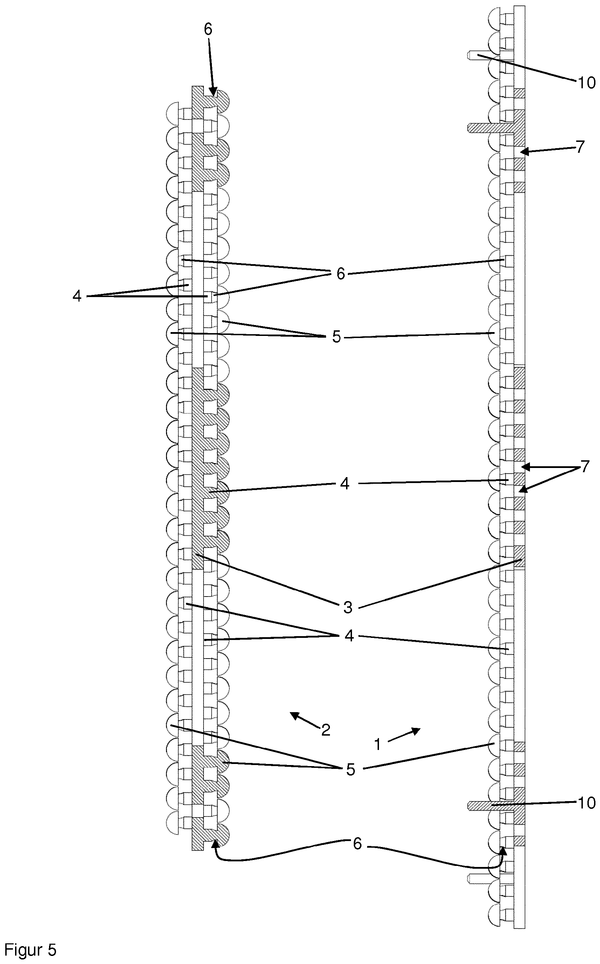

Die Figuren 1 bis 5 zeigen eine erste Ausführungsform der vorliegenden Erfindung, dabei zeigt Figur 1 eine schematische perspektivische Ansicht auf einen erfindungsgemäßen Käfig, der aus fünf Platten 1, 2 eines erfindungsgemäßen Bausatzes aufgebaut ist, Figur 2 eine schematische perspektivische Ansicht auf zwei der Platten 1, 2 nach Figur 1, die nicht miteinander gerastet sind, Figur 3 eine schematische Querschnittansicht durch die fünf gerasteten Platten 1, 2 des Käfigs nach Figur 1, Figur 4 eine vergrößerte Darstellung einer schematischen Querschnittansicht durch die fünf gerasteten Platten 1, 2 des Käfigs nach Figur 1 und Figur 5 eine schematische Querschnittansicht durch die zwei nicht gerasteten Platten nach Figur 2.

Der Bausatz weist zumindest zwei äußere Platten 1 auf, die zum Befestigen an Rücken-Wirbelkörperkörpern (nicht gezeigt) vorgesehen sind, und weist mehrere mittlere oder innere Platten 2 auf, die zwischen den äußeren Platten 1 eingesetzt werden können, um die Höhe des Käfigs einzustellen. Die Platten 1, 2 bestehen aus einem elastischen biokompatiblen Kunststoff oder aus Edelstahl, Titan, einer Titanlegierung, Tantal, einer Tantallegierung können aber auch aus Kompositen dieser Materialien gefertigt sein. Die Platten 1, 2 werden mit einem CAM-Verfahren (CAM - Computer-Aided Manufacturing, Deutsch: rechner-unterstützte Fertigung) beziehungsweise einem 3D-Druckverfahren hergestellt, beispielsweise mit selektivem Laserschmelzen SLM (Selective Laser Melting). Andere Rapid-Prototyping-Verfahren beziehungsweise computergestützte generative Herstellungsverfahren können auch zur Herstellung der Platten 1, 2 verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, Stereolithografie (STL oder SLA) von Kunststoffen, Schleif- oder Mehr-Achs-Fräsverfahren oder Digital Light Processing (DLP) von photopolymerisierbaren flüssigen Kunststoffen.

Die Platten 1, 2 weisen jeweils ein plattenförmiges Flächengebilde 3 auf, das die gesamten Platten 1, 2 trägt und in sich verbindet. Das Flächengebilde 3 kann in begrenztem Umfang flexibel und elastisch verformbar sein, so dass auch andere Flächen als Ebenen mit dem Flächengebilde 3 geformt werden können, so dass die Platten 1, 2 in geringem Umfang an die Form der Wirbelkörper angepasst werden kann. Von den Flächengebilden 3 erstrecken sich bei jeder Platte 1, 2 eine Vielzahl von Stiften 4 weg, die senkrecht von der Ebene der Flächengebilde 3 abstehen.

In den Figuren 1 bis 5 sind zwei verschiedene Typen von Platten 1, 2 dargestellt nämlich äußere Platten 1, die eine flache Unterseite aufweisen und bei denen sich die Stifte 4 nur auf einer Seite der Flächengebilde 3 von dem Flächengebilde 3 aus erstrecken, und zweitens mittlere Platten 2 beziehungsweise innere Platten 2, bei denen sich die Stifte 4 von beiden Seiten des Flächengebildes 3 aus erstrecken. Bei einem fertig aufgebauten Käfig (siehe Figuren 1, 3 und 4) sind die Platten 1, 2 sandwichartig zueinander angeordnet, wobei die äußeren Platten 1 die beiden Deckflächen bilden und die inneren Platten 2 zwischen den äußeren Platten 1 angeordnet sind. Die äußeren Platten 1 können großflächig anliegend auf den zu behandelnden Wirbelkörpern befestigt werden. Hierzu können Ösen (nicht gezeigt) in dem Flächengebilde 3 vorgesehen sein, so dass die äußeren Platten 1 auf die Wirbelkörper geschraubt werden können, oder es können auf der Seite des Flächengebildes 3 ohne Stifte 4 Spitzen (nicht gezeigt) vorgesehen sein, die in den Knochen der Wirbelkörper eingesteckt werden. Die inneren Platten 2 können allerdings theoretisch ebenfalls auf den zu behandelnden Wirbelkörpern befestigt werden, wenn auch mit einer geringeren Auflagefläche, so dass theoretisch auf die äußeren Platten 1 verzichtet werden kann.

An den ansonsten zylindrischen Stiften 4 sind an dem den Flächengebilden 3 gegenüberliegenden Enden der Stifte 4 Pilze 5 als Rastelemente 5 vorgesehen. Die Pilze 5 sind nach außen (von dem Flächengebilde 3 weg) abgerundet und bilden Kugelabschnitte. Es sind aber auch andere Abrundungen, wie beispielsweise Ellipsenabschnitte möglich. An der zum Flächengebilde 3 hin ausgerichteten Seite bilden die Pilze 5 eine ebene Greiffläche, die zum Verhaken und Rasten mit anderen Pilzen 5 eingreifender Platten 1, 2 geeignet sind.

In den Stiften 4 sind zudem angrenzend zu den Pilzen 5 beziehungsweise den Greifflächen Nuten 6 als Gegenrastmittel 6 vorgesehen, in die die Pilze 5 benachbarter Platten 1, 2 eingreifen beziehungsweise einrasten können. Hierzu sind die dem Flächengebilde 3 zugewandten Kanten der Nuten 6 abgerundet geformt, damit die Pilze 5 gut in die Nuten 6 passen beziehungsweise rasten. Die Form der Nuten 6 entspricht einem Negativ der Form der Oberfläche der Pilze 5, so dass sich diese entlang einer Linie in einer der Nuten 6 anlegen können. Die Pilze 5 bilden so Rastelemente 5 und die Nuten 6 die passenden Gegenrastmittel 6. Ein weiteres Einschieben der Platte 1, 2 nach der Rastung wird durch diesen Aufbau verhindert.

Die Figuren 3 und 4 zeigen hierzu eine schematische Querschnittansicht auf über die Pilze 5 miteinander gerastete Platten 1, 2 des erfindungsgemäßen Bausatzes.

In dem Flächengebilde 3 der äußeren Platten 1 sind zwischen den Stiften 4 eine Vielzahl von durchgehenden Poren 7 angeordnet, die eine offene Porosität des Käfigs an der Auflagefläche zum Wirbelkörper in einer Richtung senkrecht zu den Flächengebilden 3 bewirken. Hierdurch kann der Knochen der Wirbelkörper leichter mit den äußeren Platten 1 des Käfigs verwachsen.

Die Stifte 4 mit den Pilzen 5 können in Gruppen beziehungsweise Inseln von Stiften 4 beziehungsweise Pilzen 5 angeordnet sein (in den Figuren 1 bis 5 nicht gezeigt). Dadurch können sich die am Rand der Gruppen beziehungsweise Inseln angeordneten Stifte 4 leichter nach außen verbiegen, wenn die Pilze 5 einer anderen Platte 1, 2 aufgedrückt werden. Dadurch sind die Platten 1, 2 leichter miteinander verbindbar, da sich die elastischen Verformungen der Stifte 4 beim Einrasten der Pilze 5 in die Nuten 6 nicht gegenseitig behindern.

Um einen erfindungsgemäßen Käfig mit Hilfe eines erfindungsgemäßen Bausatzes aufzubauen, liegen die Platten 1, 2 bevorzugt aneinander an, liegen aber nicht miteinander verhakt oder gerastet vor, so dass also die Pilze 5 der Stifte 4 benachbarter Platten 1, 2 noch nicht ineinander greifen. Zudem können die Platten 1, 2 mit einer Flüssigkeit benetzt vorliegen. In der Flüssigkeit ist bevorzugt zumindest eine pharmazeutisch wirksame Substanz enthalten, die zur Bekämpfung einer Infektion oder zum Anregen des Knochenwachstums geeignet ist. Alternativ oder zusätzlich können die Platten 1, 2 mit einer solchen pharmazeutisch wirksamen Substanz beschichtet sein.

Der Käfig kann geformt werden, indem die Platten 1, 2 über ihre Flächen ineinander gedrückt werden. Dadurch rasten die Platten 1, 2 miteinander und der Käfig wird in der gewünschten Form verfestigt. Zuvor oder während dessen können die Platten 1, 2 auch durch eine leichte elastische Verformung der Flächengebilde 3 verformt und an die Behandlungssituation angepasst werden. Nach dem Rasten mit zumindest einer weiteren (meist dann ebenfalls verformten) inneren Platte 2 stabilisieren sich die beiden so miteinander verbundenen Platten 1, 2 gegenseitig, so dass die gewählte Form verfestigt ist.

Die Platten 1, 2 können dabei mit einander rasten, indem die Pilze 5 die Stifte 4 verbundener Platten 1, 2 elastisch verformen und durch die elastische Rückstellkraft der Stifte 4 die Pilze 5 beziehungsweise die Ränder der Pilze 5 in die Nuten 6 drücken und dadurch die Bewegung benachbarter Platten 1, 2 von dem Flächengebilde 3 weg begrenzen (siehe Figuren 3 und 4). Durch die angepasste Form der Nuten 6 an die Pilze 5 ist eine weitere Bewegung der Pilze 5 blockiert, insbesondere dann, wenn eine große Zahl von Pilzen 5 in einer großen Zahl von Nuten 6 eingerastet ist.

Bevorzugt werden die Abmessungen der Pilze 5, die Dicke des Flächengebildes 3, die Form der Nuten 6 und die Länge der Stifte 4 zwischen dem Flächengebilde 3 und den Pilzen 5 so aufeinander abgestimmt, dass bei einem Verbinden der Platten 1, 2 die von dem Flächengebilde 3 abgewandten Oberflächen der Pilze 5 an der Oberfläche der Nuten 6 benachbarter Platten 1, 2 anliegen und/oder bei einem Verbinden der Platten 1, 2 die Greifflächen der Pilze 5 in die Greifflächen der Pilze 5 der benachbarten Platte 1, 2 anliegen. Hierdurch wird erreicht, dass die verbundenen Platten 1, 2 nicht beziehungsweise nicht ohne große Krafteinwirkung gegeneinander beweglich sind.

Die Nuten 6 verhindern auch, dass die Greifflächen oder die gegenüberliegende Kappenoberseiten der Pilze 5 die Poren 7 komplett abdecken. Damit die Ausnehmungen 7 noch schlechter von den Pilzen 5 abgedeckt werden können, können die Ausnehmungen 7 mehrere Schlitze aufweisen (nicht gezeigt), die über den Umfang der Ausnehmungen 7 verteilte sind.

Der fertig aufgebaute Käfig weist zwei offene axiale Hohlräume 8 auf, die durch übereinander platzieren der Platten 1, 2 entstehen, in denen hierzu passende Ausnehmungen 9 vorhanden sind. Die offenen axialen Hohlräume 8 und damit die Ausnehmungen 9 dienen dazu, dass Knochen der Wirbelkörper sie durchwachsen kann. Dazu sind die Oberflächen der offenen axialen Hohlräume 8 und der Ausnehmungen 9 mit autologem Knochenersatzmaterial gefüllt und, wenn gewünscht, zusätzlich mit einer das Knochenwachstum fördernden Substanz beschichtet. Der freie Querschnitt der Ausnehmungen 9 und der offenen axialen Hohlräume 8 beträgt etwa 10 mm aber zumindest 5 mm, damit der Knochen der Wirbelkörper gut einwachsen kann. Die offenen axialen Hohlräume 8 bilden somit das Innere des Käfigs.

Damit die offenen axialen Hohlräume 8 gleichmäßig sind und die äußere Form des Käfigs eben ist, müssen die Platten 1, 2 fluchtend beziehungsweise passend aufeinander gerastet werden. Um dies zu erleichtern, sind jeweils vier Positionierstifte 10 als Positionierhilfen 10 auf den Flächengebilden 3 der äußeren Platten 1 vorgesehen. Dementsprechend weisen die inneren Platten 2 hierzu vier passende Bohrungen in der Flächengebilden 3 auf, so dass die Positionierstifte 10 beim rasten der Platten 1, 2 durch diese Bohrungen gesteckt werden und damit die Orientierung und Lage der inneren Platte 2 relativ zur äußeren Platte 1 vorgeben. Dadurch kann sichergestellt werden, dass die Platten 1, 2 fluchtend beziehungsweise passend aufeinander platziert werden. Die Platten 1, 2 haben alle bezogen auf die Ebene der Flächengebilde 3 die gleiche Form, so dass sie passend aufeinander gelegt werden können. Bei der in den Figuren 1 bis 5 gezeigten Ausführung ist die Länge der Positionierstifte 10 gerade so gewählt, dass sie nur durch die Bohrung der benachbarten Platte 2 reicht. Es wäre aber ohne weiteres möglich, längere Positionierstifte 10 vorzusehen, die durch die Bohrungen weiterer Platten 1, 2 reicht. Ebenso könnten die Positionierstifte 10 auch an einer inneren Platte 2 vorzusehen und in den äußeren Platten 1 Bohrungen vorzusehen. Die Positionierstifte 10 könnten dann auch in Spitzen enden, die in den Wirbelkörper eingesteckt und darin befestigt werden. Ebenso können bei dieser Variante (nicht gezeigt) die Positionierstifte 10 sich in beide Richtungen senkrecht von dem Flächengebilde erstrecken.

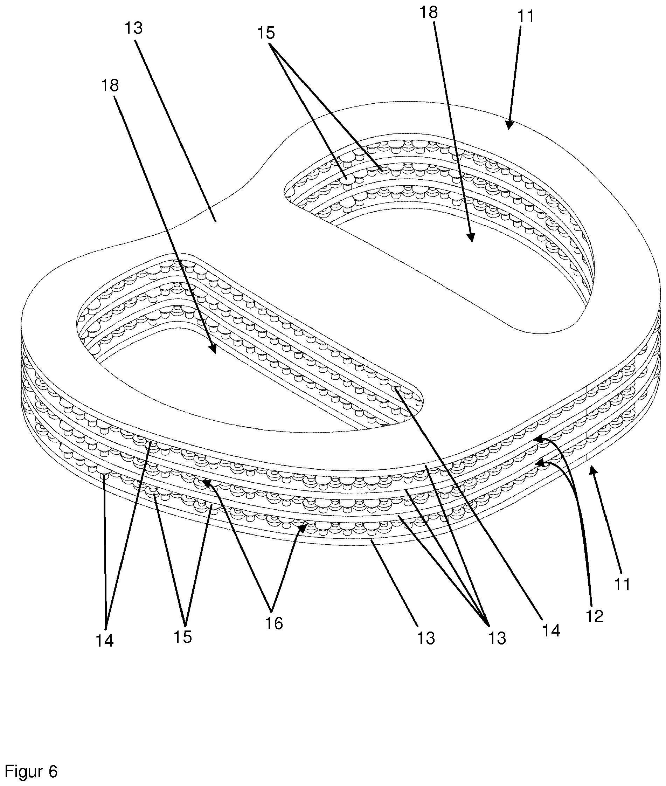

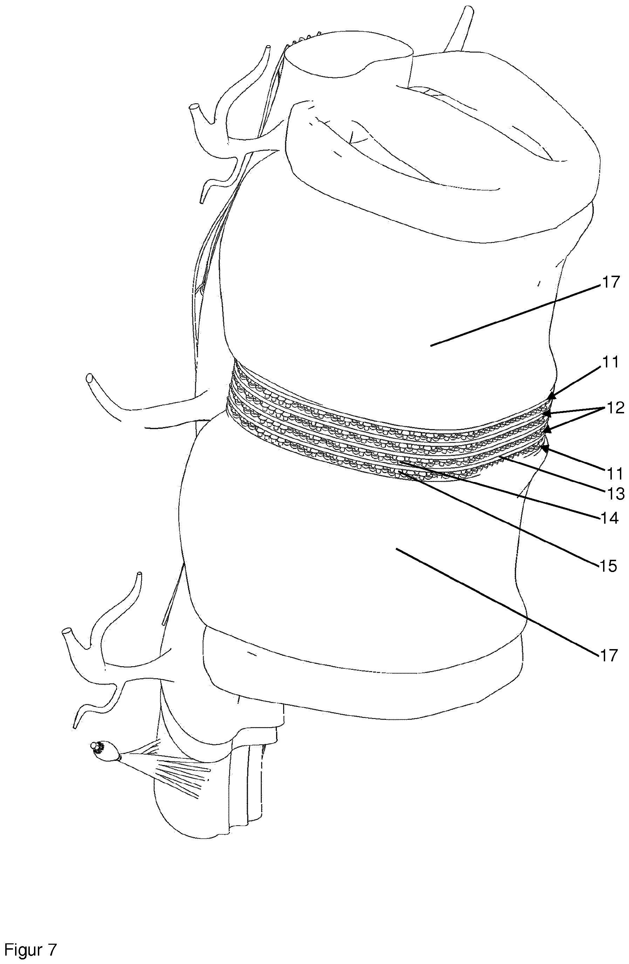

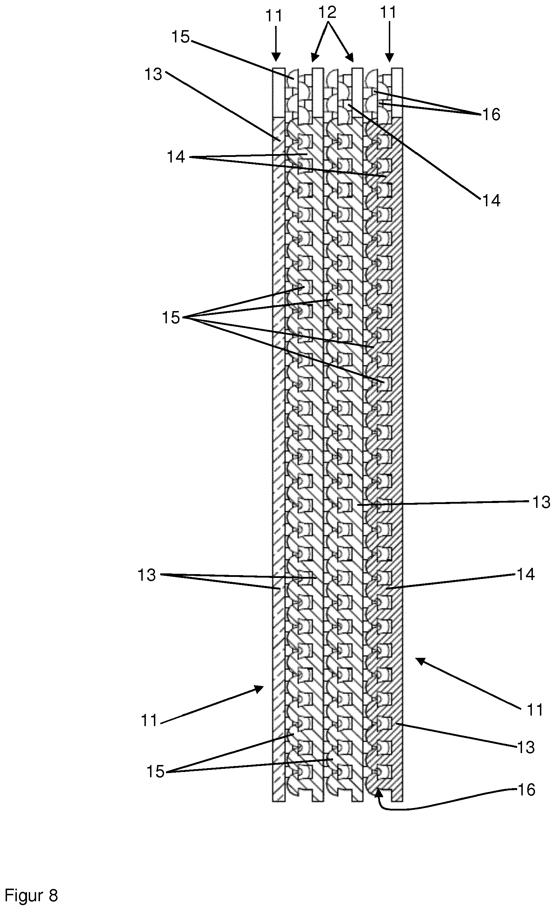

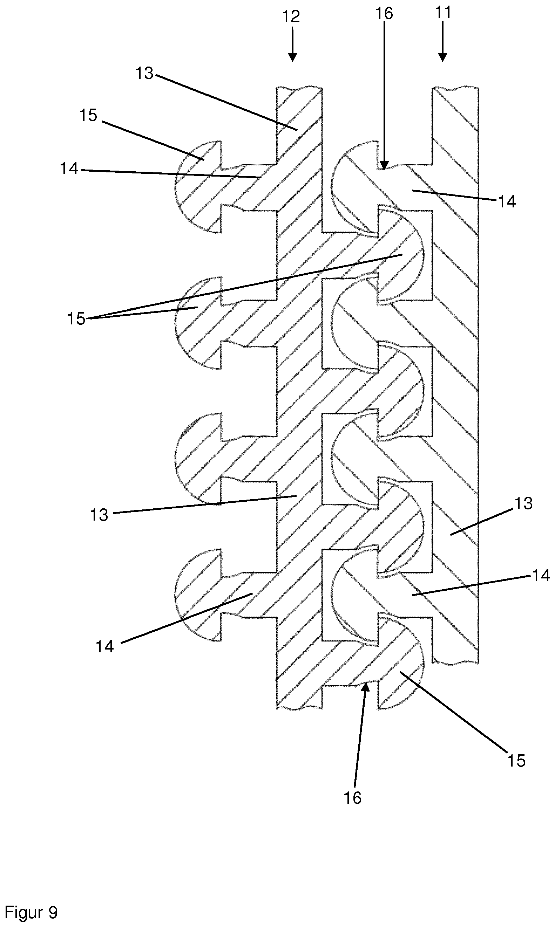

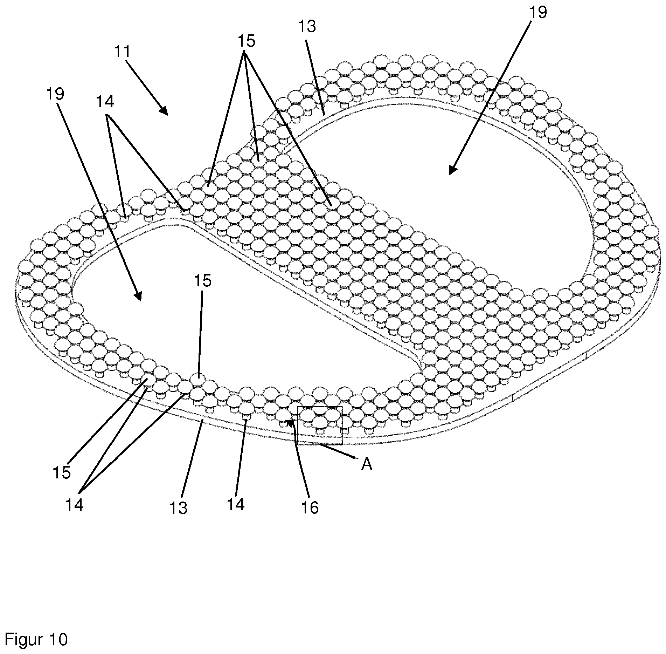

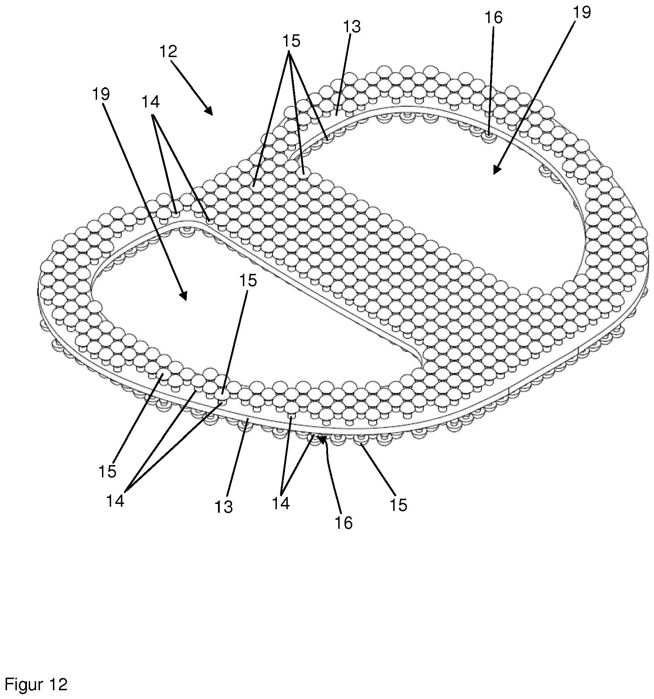

Die Figuren 6 bis 12 zeigen eine zweite Ausführungsform der vorliegenden Erfindung, dabei zeigt Figur 6 eine schematische perspektivische Ansicht auf einen zweiten erfindungsgemäßen Käfig, der aus vier Platten 11, 12 eines zweiten erfindungsgemäßen Bausatzes aufgebaut ist, Figur 7 eine schematische perspektivische Ansicht des Käfigs nach Figur 6 im beim Patienten eingesetzten Zustand, Figur 8 eine schematische Querschnittansicht durch die vier gerasteten Platten 11, 12 des Käfigs nach Figur 6, Figur 9 eine vergrößerte Darstellung einer schematischen Querschnittansicht durch zwei gerastete Platten 11, 12 des Bausatzes nach den Figuren 6 bis 8, Figur 10 eine schematische perspektivische Ansicht auf eine äußere Platten 11 nach einer der Figuren 6 bis 9, Figur 11 eine Vergrößerung eines Teilbereichs A in Figur 10 und Figur 12 eine schematische perspektivische Ansicht auf eine der inneren Platten 12 nach einer der Figuren 6 bis 9.

Der Bausatz weist zumindest zwei äußere Platten 11 auf, die zum Befestigen an Rücken-Wirbelkörpern 17 (siehe Figur 7) vorgesehen sind, und weist mehrere mittlere oder innere Platten 12 auf, die zwischen den äußeren Platten 11 eingesetzt werden können, um die Höhe des Käfigs einzustellen. Die Platten 11, 12 bestehen aus einem elastischen biokompatiblen Kunststoff oder aus Edelstahl, Titan, einer Titanlegierung, Tantal, einer Tantallegierung können aber auch aus Kompositen dieser Materialien gefertigt sein. Die Platten 11, 12 werden mit einem CAM-Verfahren (CAM - Computer-Aided Manufacturing, Deutsch: rechner-unterstützte Fertigung) beziehungsweise einem 3D-Druckverfahren hergestellt, beispielsweise mit selektivem Laserschmelzen SLM (Selective Laser Melting). Andere Rapid-Prototyping-Verfahren beziehungsweise computergestützte generative Herstellungsverfahren können auch zur Herstellung der Platten 11, 12 verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, Stereolithografie (STL oder SLA) von Kunststoffen, Schleif- oder Mehr-Achs-Fräsverfahren oder Digital Light Processing (DLP) von photopolymerisierbaren flüssigen Kunststoffen.

Die Platten 11, 12 weisen jeweils ein plattenförmiges Flächengebilde 13 auf, das die gesamten Platten 11, 12 trägt und in sich verbindet. Das Flächengebilde 13 kann in begrenztem Umfang flexibel und elastisch verformbar sein, so dass auch andere Flächen als Ebenen mit dem Flächengebilde 13 geformt werden können, so dass die Platten 11, 12 in geringem Umfang an die Form der Wirbelkörper 17 angepasst werden kann. Von den Flächengebilden 13 erstrecken sich bei jeder Platte 11, 12 eine Vielzahl von Stiften 14 weg, die senkrecht von der Ebene der Flächengebilde 13 abstehen.

In den Figuren 6 bis 12 sind zwei verschiedene Typen von Platten 11, 12 dargestellt nämlich äußere Platten 11, die eine flache Unterseite aufweisen und bei denen sich die Stifte 14 nur auf einer Seite der Flächengebilde 13 von dem Flächengebilde 13 aus erstrecken, und zweitens mittlere Platten 12 beziehungsweise innere Platten 12, bei denen sich die Stifte 14 von beiden Seiten des Flächengebildes 13 aus erstrecken. Bei einem fertig aufgebauten Käfig (siehe Figuren 6, 7 und 8) sind die Platten 11, 12 sandwichartig zueinander angeordnet, wobei die äußeren Platten 11 die beiden Deckflächen bilden und die inneren Platten 12 zwischen den äußeren Platten 11 angeordnet sind. Die äußeren Platten 11 können großflächig anliegend auf den zu behandelnden Wirbelkörpern 17 befestigt werden. Hierzu können Ösen (nicht gezeigt) in dem Flächengebilde 13 vorgesehen sein, so dass die äußeren Platten 11 auf die Wirbelkörper 17 geschraubt werden können, oder es können auf der Seite des Flächengebildes 13 ohne Stifte 14 Spitzen (nicht gezeigt) vorgesehen sein, die in den Knochen der Wirbelkörper 17 eingesteckt werden. Die inneren Platten 12 können allerdings theoretisch ebenfalls auf den zu behandelnden Wirbelkörpern befestigt werden, wenn auch mit einer geringeren Auflagefläche, so dass theoretisch auf die äußeren Platten 11 verzichtet werden kann.

An den ansonsten zylindrischen Stiften 14 sind an dem den Flächengebilden 13 gegenüberliegenden Enden der Stifte 14 Pilze 15 als Rastelemente 15 vorgesehen. Die Pilze 15 sind nach außen (von dem Flächengebilde 13 weg) abgerundet und bilden Kugelabschnitte. Es sind aber auch andere Abrundungen, wie beispielsweise Ellipsenabschnitte möglich. An der zum Flächengebilde 13 hin ausgerichteten Seite bilden die Pilze 15 eine ebene Greiffläche, die zum Verhaken und Rasten mit anderen Pilzen 15 eingreifender Platten 11, 12 geeignet sind.

In den Stiften 14 sind zudem angrenzend zu den Pilzen 15 beziehungsweise den Greifflächen Nuten 16 als Gegenrastmittel 16 vorgesehen, in die die Pilze 15 benachbarter Platten 11, 12 eingreifen beziehungsweise einrasten können. Hierzu sind die dem Flächengebilde 13 zugewandten Kanten der Nuten 16 abgerundet geformt, damit die Pilze 15 gut in die Nuten 16 passen beziehungsweise rasten. Die Form der Nuten 16 entspricht einem Negativ der Form der Oberfläche der Pilze 15, so dass sich diese entlang einer Linie in einer der Nuten 16 anlegen können. Die Pilze 15 bilden so Rastelemente 15 und die Nuten 16 die passenden Gegenrastmittel 16. Ein weiteres Einschieben der Platte 11, 12 nach der Rastung wird durch diesen Aufbau verhindert.

Die Figuren 8 und 9 zeigen hierzu eine schematische Querschnittansicht auf über die Pilze 15 miteinander gerastete Platten 11, 12 des erfindungsgemäßen Bausatzes. In dem Flächengebilde 13 im Gegensatz zum ersten Ausführungsbeispiel keine Poren angeordnet.

Die Stifte 14 mit den Pilzen 15 können in Gruppen beziehungsweise Inseln von Stiften 14 beziehungsweise Pilzen 15 angeordnet sein (in den Figuren 1 bis 5 nicht gezeigt). Dadurch können sich die am Rand der Gruppen beziehungsweise Inseln angeordneten Stifte 14 leichter nach außen verbiegen, wenn die Pilze 15 einer anderen Platte 11, 12 aufgedrückt werden. Dadurch sind die Platten 11, 12 leichter miteinander verbindbar, da sich die elastischen Verformungen der Stifte 14 beim Einrasten der Pilze 15 in die Nuten 16 nicht gegenseitig behindern.

Um einen erfindungsgemäßen Käfig mit Hilfe eines erfindungsgemäßen Bausatzes aufzubauen, liegen die Platten 11, 12 bevorzugt aneinander an, liegen aber nicht miteinander verhakt oder gerastet vor, so dass also die Pilze 15 der Stifte 14 benachbarter Platten 11, 12 noch nicht ineinander greifen. Zudem können die Platten 11, 12 mit einer Flüssigkeit benetzt vorliegen. In der Flüssigkeit ist bevorzugt zumindest eine pharmazeutisch wirksame Substanz enthalten, die zur Bekämpfung einer Infektion oder zum Anregen des Knochenwachstums geeignet ist. Alternativ oder zusätzlich können die Platten 11, 12 mit einer solchen pharmazeutisch wirksamen Substanz beschichtet sein.

Der Käfig kann geformt werden, indem die Platten 11, 12 über ihre Flächen ineinander gedrückt werden. Dadurch rasten die Platten 11, 12 miteinander und der Käfig wird in der gewünschten Form verfestigt. Zuvor oder während dessen können die Platten 11, 12 auch durch eine leichte elastische Verformung der Flächengebilde 13 verformt und an die Behandlungssituation angepasst werden. Nach dem Rasten mit zumindest einer weiteren (meist dann ebenfalls verformten) inneren Platte 12 stabilisieren sich die beiden so miteinander verbundenen Platten 11, 12 gegenseitig, so dass die gewählte Form verfestigt ist.

Die Platten 11, 12 können dabei mit einander rasten, indem die Pilze 15 die Stifte 14 verbundener Platten 11, 12 elastisch verformen und durch die elastische Rückstellkraft der Stifte 14 die Pilze 15 beziehungsweise die Ränder der Pilze 15 in die Nuten 16 drücken und dadurch die Bewegung benachbarter Platten 11, 12 von dem Flächengebilde 13 weg begrenzen (siehe Figuren 8 und 9). Durch die angepasste Form der Nuten 16 an die Pilze 15 ist eine weitere Bewegung der Pilze 15 blockiert, insbesondere dann, wenn eine große Zahl von Pilzen 15 in einer großen Zahl von Nuten 16 eingerastet ist.

Bevorzugt werden die Abmessungen der Pilze 15, die Dicke des Flächengebildes 13, die Form der Nuten 16 und die Länge der Stifte 14 zwischen dem Flächengebilde 13 und den Pilzen 15 so aufeinander abgestimmt, dass bei einem Verbinden der Platten 11, 12 die von dem Flächengebilde 13 abgewandten Oberflächen der Pilze 15 an der Oberfläche der Nuten 16 benachbarter Platten 11, 12 anliegen und/oder bei einem Verbinden der Platten 11, 12 die Greifflächen der Pilze 15 in die Greifflächen der Pilze 15 der benachbarten Platte 11, 12 anliegen. Hierdurch wird erreicht, dass die verbundenen Platten 11, 12 nicht beziehungsweise nicht ohne große Krafteinwirkung gegeneinander beweglich sind.

Der fertig aufgebaute Käfig weist zwei offene axiale Hohlräume 18 auf, die durch übereinander platzieren der Platten 11, 12 entstehen, in denen hierzu passende Ausnehmungen 19 vorhanden sind. Die offenen axialen Hohlräume 18 und damit die Ausnehmungen 19 dienen dazu, dass Knochen der Wirbelkörper 17 sie durchwachsen kann. Dazu sind die Oberflächen der offenen axialen Hohlräume 18 und der Ausnehmungen 19 mit autologem Knochenersatzmaterial gefüllt und, wenn gewünscht, zusätzlich mit einer das Knochenwachstum fördernden Substanz beschichtet. Der freie Querschnitt der Ausnehmungen 19 und der offenen axialen Hohlräume 18 beträgt etwa12 mm aber zumindest 5 mm, damit der Knochen der Wirbelkörper gut einwachsen kann. Die offenen axialen Hohlräume 18 bilden somit das Innere des Käfigs.

Damit die offenen axialen Hohlräume 18 gleichmäßig sind und die äußere Form des Käfigs eben ist, müssen die Platten 11, 12 fluchtend beziehungsweise passend aufeinander gerastet werden. Um dies zu erleichtern, könnten analog dem ersten Ausführungsbeispiel Positionierstifte (nicht gezeigt) als Positionierhilfen vorgesehen sein. Die Platten 11, 12 haben alle bezogen auf die Ebene der Flächengebilde 13 die gleiche Form, so dass sie passend aufeinander gelegt werden können.

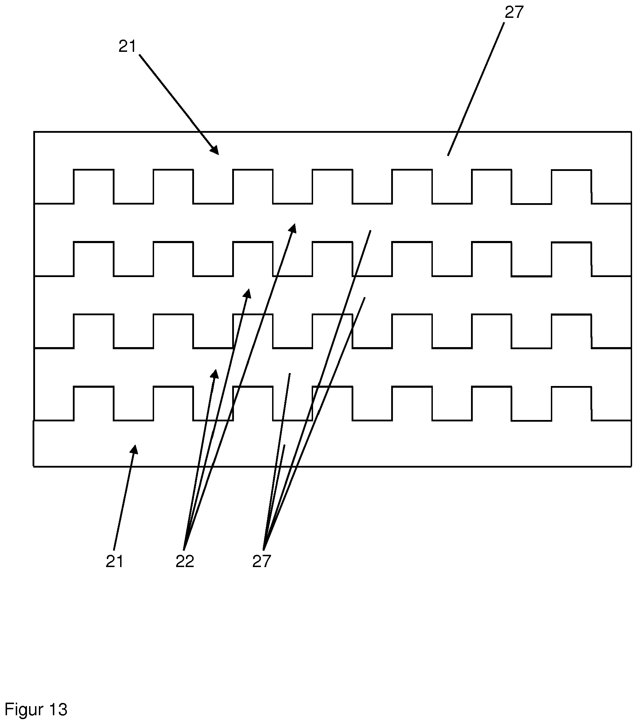

Figur 13 zeigt eine schematische Seitenansicht auf fünf miteinander verzahnte Platten 21, 22, die einen dritten erfindungsgemäßen Käfig bilden. Der Käfig ist analog zu einem der ersten beiden Ausführungsbeispiele ausgeführt. Im Gegensatz zu diesen Ausführungsbeispielen sind an den Flächengebilden (in Figur 13 nicht zu sehen) der äußeren Platten 21 und der inneren Platten 22 außen umlaufende Ränder 27 vorgesehen. Die Ränder 27 liegen verzahnt aneinander an, wenn die Platten 21, 22 miteinander gerastet sind. Hierdurch wird der Käfig nach außen abgeschlossen, so dass autologes Knochenmaterial aus dem Inneren des Käfigs nicht nach außen dringen kann. Der Käfig kann durch Poren in den Platten 21, 22 im inneren offenporig ausgeführt sein, so dass eine gute Durchwachsung mit Knochen möglich ist, ohne dass Materialien hierzu aus dem Inneren des Käfigs nach außen gelangen können.

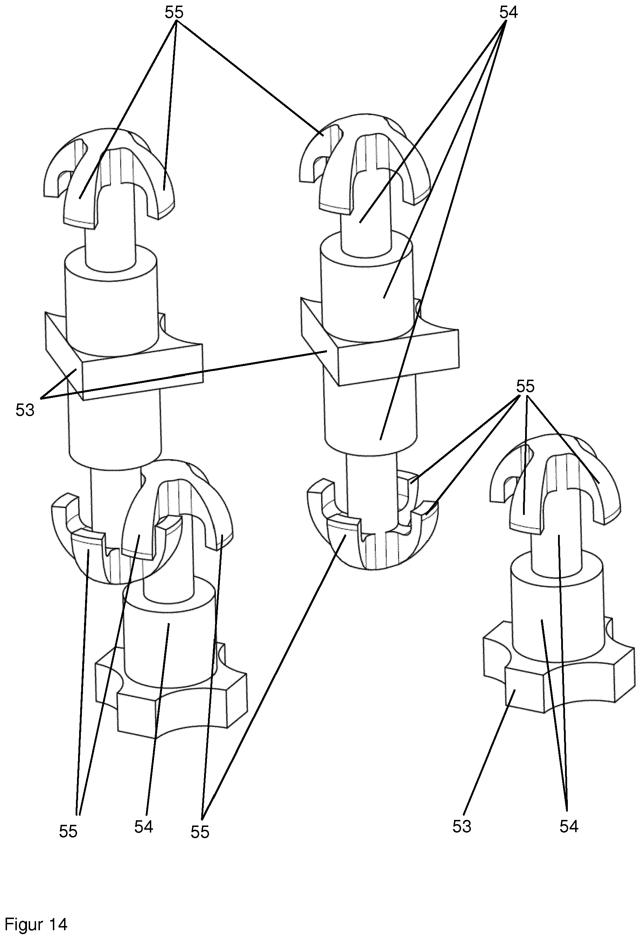

Figur 14 zeigt eine schematische perspektivische Detailansicht auf zwei miteinander verhakte Haken 55 (links) zwei nicht miteinander verhakte Haken 55 (rechts) als Teile von Platten eines vierten erfindungsgemäßen Bausatzes.

Der Aufbau der vollständigen Platten hierzu ist analog der Platten gemäß einem der vorherigen Ausführungsbeispiele, wobei anstatt Pilze die Haken 55 zum Verbinden der Platten vorgesehen sind.

An den ansonsten zylindrischen Stiften 54 sind an dem den Flächengebilden 53 gegenüberliegenden Enden der Stifte 54 Haken 55 als Rastelemente 55 vorgesehen. Die Haken 55 sind nach außen (von dem Flächengebilde 53 weg) abgerundet und bilden Teile von Kugeloberflächen. Es sind aber auch andere Abrundungen, wie beispielsweise Ellipsenabschnitte möglich. An der zum Flächengebilde 53 hin ausgerichteten Seite bilden die Haken 55 Hinterschnitte, die zum Verhaken oder Rasten mit anderen Haken 55 eingreifender Platten geeignet sind.

Die Stifte 54 sind im Bereich angrenzend zu den Haken 55 beziehungsweise den Hinterschnitten der Haken 55 dünner beziehungsweise mit einem geringeren Querschnitt (als Nuten) ausgeformt. In die dünneren Bereiche können die Haken 55 benachbarter Platten leichter eingreifen beziehungsweise einrasten.

Bei der vorliegenden vierten Ausführungsform sind ausschließlich Haken 55 als Rastelemente 55 vorgesehen. Um einen erfindungsgemäßen Käfig zu bilden, werden die Platten aneinander anliegend aber nicht miteinander verhakt oder gerastet verwendet, so dass also die Haken 55 der Stifte 54 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung einer Infektion oder zum Anregen des Knochenwachstums geeignet ist. Alternativ oder zusätzlich können die Platten mit einer solchen pharmazeutisch wirksamen Substanz beschichtet sein.

Der Käfig kann geformt werden, indem die Platten über ihre Flächen ineinander gedrückt werden. Dadurch verhaken oder rasten die Platten miteinander und der Käfig wird in der gewünschten Form verfestigt.

Die Platten verbinden sich dabei untereinander in der Art, dass freie Zwischenräume zwischen den miteinander verbundenen Platten im Bereich der Stifte 54 und der Haken 55 verbleiben, so dass der aus den Platten geformte Käfig in den Richtungen parallel zur Ebene der Platten offenporig ist. Die Platten haben einen Querschnitt beziehungsweise eine Dicke von etwa 5 mm, so dass die verbleibenden Poren einen freien Querschnitt im Bereich von etwa 0,5 mm aufweisen. Dieser Querschnitt reicht aus, damit sich in den Poren Knochenmaterial ausbilden beziehungsweise einwachsen kann. Der Käfig mit seinen offenen Poren kann also als osteokonduktiv bezeichnet werden. Der aus den Platten geformte Käfig ist daher zur Verbindung mit den Wirbelkörpern gut geeignet.

Die Platten sollten fest ineinander gedrückt werden, so dass der Käfig formstabil ist. Die Platten können dabei in einer ersten Stufe mit einander rasten, indem die Haken 55 die Stifte 54 verbundener Platten elastisch verformen und durch die elastische Rückstellkraft der Stifte 54 die Haken 55 beziehungsweise Spitzen der Haken 55 ineinander drücken und dadurch die Bewegung benachbarter Platten von dem Flächengebilde 53 weg begrenzen. Hierdurch wird erreicht, dass die verbundenen Platten nicht ohne Verformung gegeneinander beweglich sind.

Die vierte Ausführungsform nach Figur 14 unterscheidet sich also von der nach den Figuren 1 bis 12 vor allem dadurch, dass Haken 55 als Rastelemente 55 vorgesehen sind.

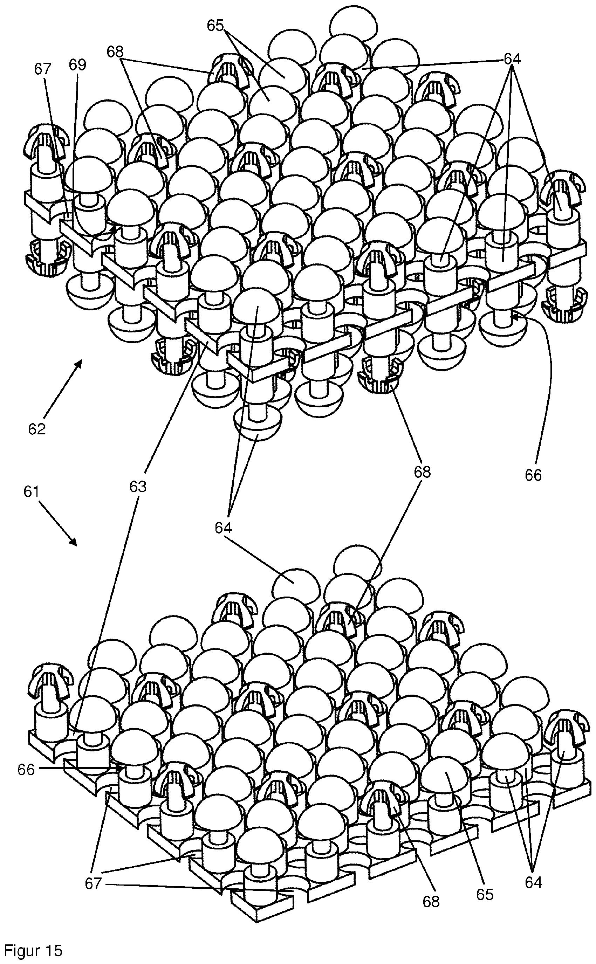

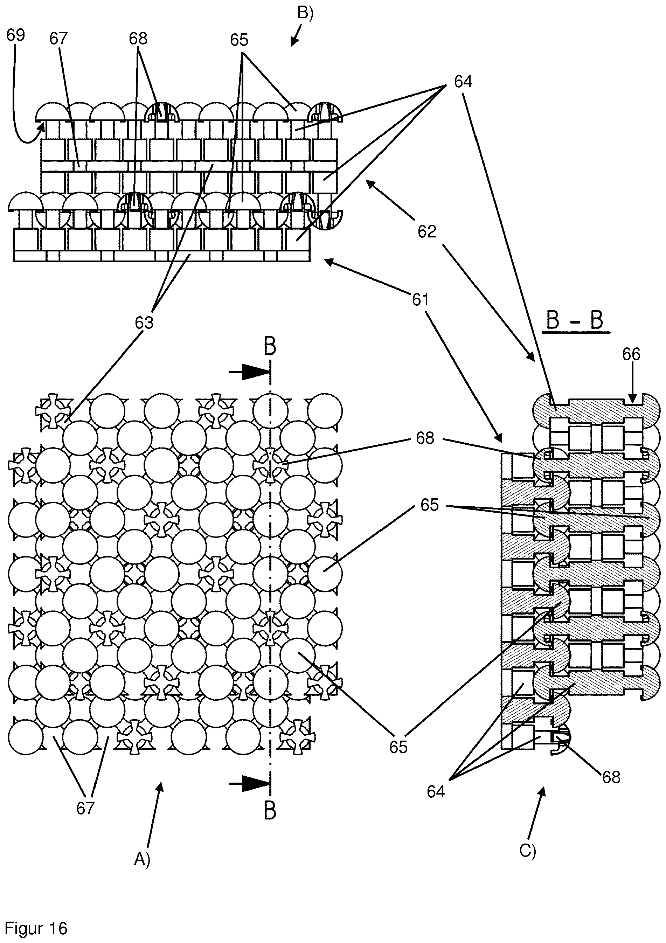

Figur 15 zeigt eine schematische perspektivische Ansicht auf Ausschnitte von zwei nicht miteinander verhakten Platten 61, 62 eines fünften erfindungsgemäßen Bausatzes und Figur 16 drei schematische Ansichten der Ausschnitte von zwei miteinander verbundenen Platten (nach Figur 15) des fünften Bausatzes A) als Aufsicht auf die Oberseite, B) in einer Seitenansicht, C) in einer Querschnittansicht entlang des Schnitts B-B nach Figur 16 A).

Die Platten 61, 62 bestehen aus einem biokompatiblen Metall, insbesondere aus Edelstahl, Titan oder einer Titanlegierung, Tantal oder einer Tantallegierung können aber auch aus einem elastischen biokompatiblen Kunststoff oder aus einem Komposit solcher Materialien gefertigt sein. Die Platten 61, 62 werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten 61, 62 verwendet werden.

Die Platten 61, 62 weisen jeweils ein plattenförmiges Flächengebilde 63 auf, das die gesamten Platten 61, 62 trägt und in sich verbindet. Das Flächengebilde 63 ist elastisch verformbar, so dass auch andere Flächen als Ebenen mit dem Flächengebilde 63 geformt werden können. Von dem Flächengebilde 63 erstrecken sich bei jeder Platte 61, 62 eine Vielzahl von Stiften 64 weg, die senkrecht von der Ebene der Flächengebilde 63 abstehen. In dem Flächengebilde 63 sind zwischen den Stiften 64 eine Vielzahl von durchgehenden Poren 67 angeordnet, die, wenn die Platten 61, 62 miteinander zu einem Käfig verbunden sind, eine offene Porosität des Käfigs in einer Richtung senkrecht zu den Flächengebilden 63 bewirken können, wenn nicht die benachbarten Platten 61, 62 anliegen und dadurch die Poren 67 abdecken.

In den Figuren 15 und 16 sind zwei verschiedene Typen von Platten 61, 62 dargestellt nämlich erstens eine äußere Platte 61, die eine flache Unterseite aufweisen und bei denen sich die Stifte 64 nur auf einer Seite der Flächengebilde 63 von dem Flächengebilde 63 aus erstrecken, und zweitens innere Platten 62, bei denen sich die Stifte 64 von beiden Seiten des Flächengebildes 63 aus erstrecken. Die äußeren Platten 61 sind in Figur 15 unten, in Figur 16 A) unten (in der Bildebene), in Figur 16 B) unten und in Figur 16 C) links gezeigt. Die inneren Platten 62 sind in Figur 15 oben und in Figur 16 oben auf, also in Figur 16 A) oben (aus der Bildebene), in Figur 16 B) oben und in Figur 16 C) rechts gezeigt. Die äußeren Platten 61 können großflächig anliegend auf den zu behandelnden Wirbelkörpern (nicht gezeigt) befestigt werden. Die inneren Platten 62 können allerdings ebenfalls auf den zu behandelnden Wirbelkörpern befestigt werden, wenn auch mit einer geringeren Auflagefläche, so dass auf die äußeren Platten 61 theoretisch verzichtet werden kann.

An den ansonsten zylindrischen Stiften 64 sind an dem den Flächengebilden 63 gegenüberliegenden Enden der Stifte 64 Pilze 65 oder Gruppen von jeweils vier Haken 68 als Rastelemente 65, 68 vorgesehen. Die Pilze 65 sind nach außen (von dem Flächengebilde 63 weg) abgerundet und bilden Kugelabschnitte. Es sind aber auch andere Abrundungen, wie beispielsweise Ellipsenabschnitte möglich. Die Haken 68 sind in gleicher Weise nach außen abgerundet. An der zum Flächengebilde 63 hin ausgerichteten Seite bilden die Pilze 65 eine ebene Greiffläche 69, die zum Verhaken oder Rasten mit anderen Pilzen 65 und Haken 68 eingreifender Platten 61, 62 geeignet sind. Dementsprechend bilden die Haken 68 an der zum Flächengebilde 63 hin ausgerichteten Seite Hinterschnitte, die zum Verhaken und Rasten mit anderen Pilzen 65 und Haken 68 eingreifender Platten 61, 62 geeignet sind.

In den Stiften 64 sind zudem angrenzend zu den Greifflächen 69 und angrenzend zu den Haken 68 Nuten 66 als Gegenrastmittel 66 vorgesehen, in die die Pilze 65 und Haken 68 benachbarter Platten 61, 62 eingreifen beziehungsweise einrasten können. Hierzu können die Nuten 66 im Gegensatz zu den gezeigten Nuten 66, aber erfindungsgemäß bevorzugt, als Negativ der Form der Wölbung der Pilze 65 beziehungsweise der Haken 68 geformt sein (analog der ersten beiden Ausführungsbeispiele), damit die Pilze 65 und Haken 68 gut in die Nuten 66 passen.

Bei der vorliegenden fünften Ausführungsform sind an den Platten 61, 62 Pilze 65 und Haken 68 gemischt als Rastelemente 65, 68 vorgesehen, wobei zwei von elf Rastelementen 65, 68 Haken 68 sind und der Rest Pilze 65. Das kann auch umgekehrt sein und die Haken 68 und Pilze 65 können auch in einem anderen Mischungsverhältnis vorliegen.

Um einen erfindungsgemäßen Käfig zu bilden, liegen die Platten 61, 62 aneinander anliegend aber nicht miteinander verhakt oder gerastet vor (also nicht so wie in Figur 16 gezeigt), so dass also die Pilze 65 und Haken 68 der Stifte 64 benachbarter Platten 61, 62 noch nicht ineinander greifen. Zudem können die Platten 61, 62 mit einer Flüssigkeit benetzt vorliegen. In der Flüssigkeit ist bevorzugt zumindest eine pharmazeutisch wirksame Substanz enthalten, insbesondere eine autologe Knochensubstanz enthalten. Alternativ oder zusätzlich können die Platten mit einer solchen pharmazeutisch wirksamen Substanz beschichtet sein.

Der Käfig kann geformt werden, indem die Platten 61, 62 über ihre Flächen ineinander gedrückt werden. Dadurch verhaken und rasten die Platten 61, 62 miteinander und der Käfig wird in der gewünschten Form verfestigt. Zuvor können die Platten 61, 62 auch durch elastische Verformung der Flächengebilde 63 verformt und an die Behandlungssituation angepasst werden. Nach dem Verhaken oder Rasten mit zumindest einer weiteren Platte 61, 62 stabilisieren sich die beiden so miteinander verbundenen Platten 61, 62 gegenseitig, so dass die gewählte Form verfestigt ist.

Die Platten 61, 62 verbinden sich dabei untereinander in der Art, dass freie Zwischenräume zwischen den miteinander verbundenen Platten 61, 62 im Bereich der Stifte 64, der Pilze 65, der Haken 68 und der Nuten 66 verbleiben, so dass der aus den Platten 61, 62 geformte Käfige in den Richtungen parallel zur Ebene der Platten 61, 62 offenporig ist. Die Platten 61, 62 haben einen Querschnitt beziehungsweise eine Dicke von etwa 9 mm, so dass die verbleibenden Poren 67 einen freien Querschnitt im Bereich von etwa 0,9 mm aufweisen. Dieser Querschnitt reicht aus, damit sich in den Poren 67 Knochenmaterial ausbilden beziehungsweise einwachsen kann. Der Käfig mit seinen offenen Poren 67 kann also als osteokonduktiv bezeichnet werden. Der aus den Platten 61, 62 geformte Käfig ist daher zum Verbinden mit dem Knochen der Wirbelkörper gut geeignet.

Die Platten 61, 62 sollten fest ineinander gedrückt werden, so dass der Käfig formstabil ist. Die Platten 61, 62 können dabei mit einander rasten, indem die Pilze 65 und Haken 68 die Stifte 64 verbundener Platten 61, 62 elastisch verformen und durch die elastische Rückstellkraft der Stifte 64 die Pilze 65 und Haken 68 beziehungsweise die Ränder der Pilze 65 und Spitzen der Haken 68 in die Nuten 66 drücken und dadurch die Bewegung benachbarter Platten 61, 62 von dem Flächengebilde 63 weg begrenzen (siehe Figur 16). Es ist auch möglich, dass erstens die Kanten der Pilze 65 und/oder die Spitzen der Haken 68 die Stifte 64, die Nuten 66 oder die Pilze 65 in geringem Umfang plastisch verformen und dadurch eine Rastung der Platten 61, 62 miteinander erfolgt.

Bevorzugt werden die Abmessungen der Pilze 65, der Haken 68, die Dicke des Flächengebildes 63, die Form der Nuten 66 und die Länge der Stifte 64 zwischen dem Flächengebilde 63 und den Pilzen 65 oder Haken 68 so aufeinander abgestimmt, dass bei einem Verbinden der Platten 61, 62 die von dem Flächengebilde 63 abgewandten Oberflächen der Pilze 65 und Haken 68 an der Oberfläche der Flächengebilde 63 benachbarter Platten 61, 62 anliegen und/oder bei einem Verbinden der Platten 61, 62 die von dem Flächengebilde 63 abgewandten Oberflächen der Pilze 65 und Haken 68 an der Greiffläche 69 der Pilze 65 und bevorzugt entlang zumindest einer Linie oder besonders bevorzugt flächig an den Nuten 66 der Stifte 64 der benachbarten Platte 61, 62 anliegen. Hierdurch wird erreicht, dass die verbundenen Platten 61, 62 nicht ohne Verformung gegeneinander beweglich sind.

Die fünfte Ausführungsform nach den Figuren 15 und 16 unterscheidet sich also von der nach den Figuren 1 bis 5 vor allem dadurch, dass Haken 68 als Rastelemente 68 vorgesehen sind.

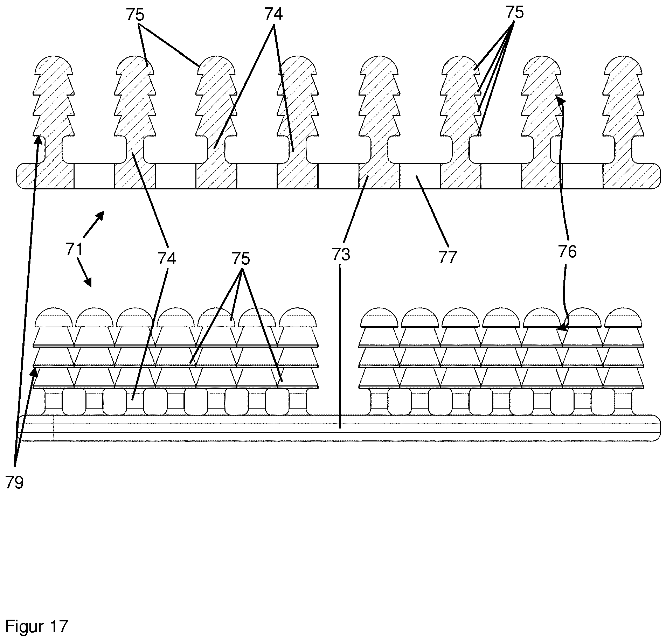

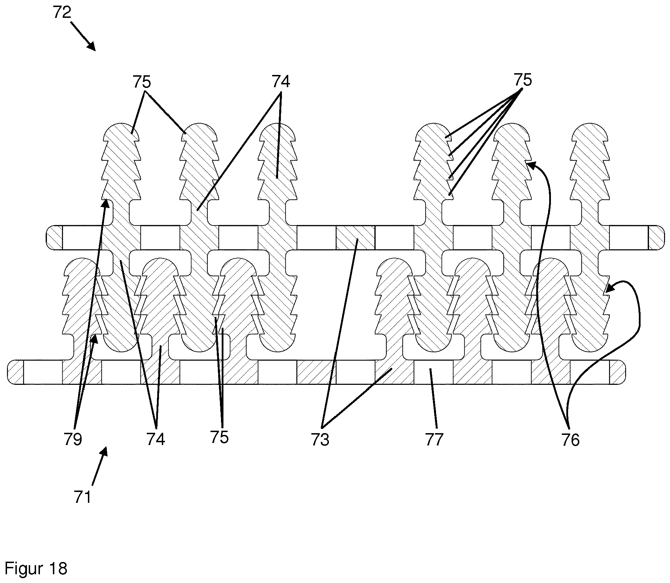

Figur 17 zeigt eine schematische Querschnittansicht durch eine äußere Platte eines sechsten erfindungsgemäßen Bausatzes (Figur 17 oben) und eine schematische Seitenansicht auf die äußere Platte des sechsten Bausatzes (Figur 17 unten). Figur 18 zeigt hierzu eine vergrößerte Darstellung einer schematischen Querschnittansicht durch zwei gerastete Platten des sechsten Bausatzes nach Figur 17.

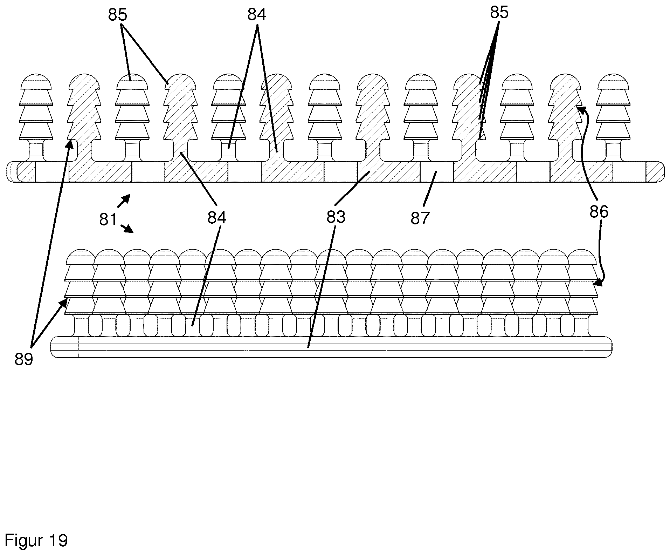

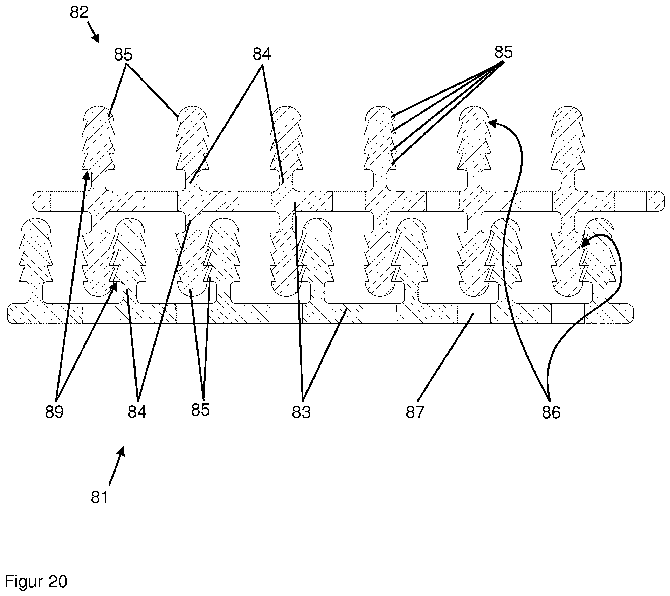

Figur 19 zeigt eine schematische Querschnittansicht durch eine äußere Platte eines siebten erfindungsgemäßen Bausatzes (Figur 19 oben) und eine schematische Seitenansicht auf die äußere Platte des siebten Bausatzes (Figur 19 unten). Figur 20 zeigt hierzu eine vergrößerte Darstellung einer schematischen Querschnittansicht durch zwei gerastete Platten des siebten Bausatzes nach Figur 19.

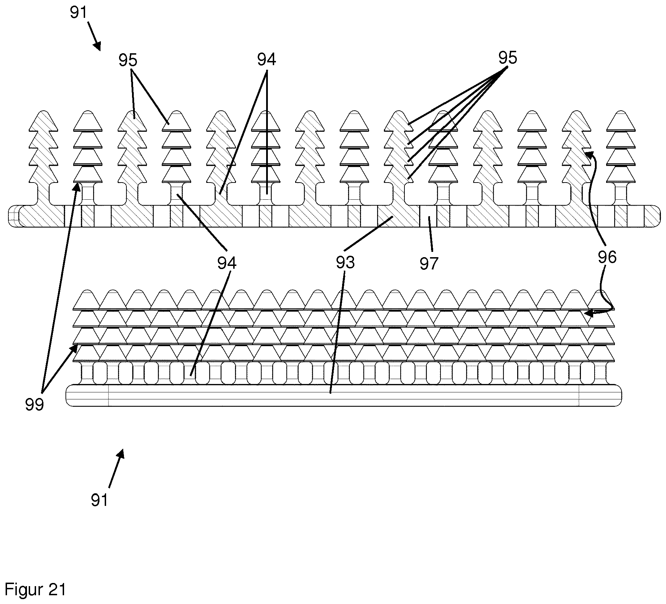

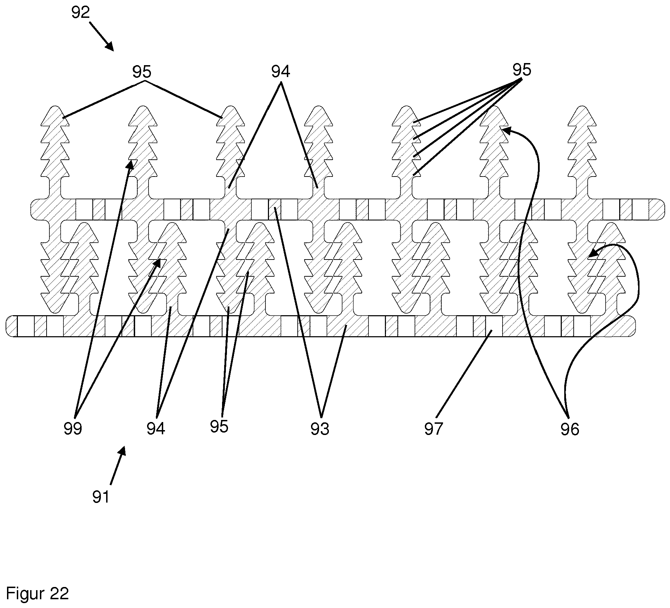

Figur 21 zeigt eine schematische Querschnittansicht durch eine äußere Platte eines achten erfindungsgemäßen Bausatzes (Figur 21 oben) und eine schematische Seitenansicht auf die äußere Platte des achten Bausatzes (Figur 21 unten). Figur 22 zeigt hierzu eine vergrößerte Darstellung einer schematischen Querschnittansicht durch zwei gerastete Platten des achten Bausatzes nach Figur 21.

Diese drei Ausführungsformen sechs, sieben und acht ähneln sich untereinander sehr stark und können daher im Folgenden gemeinsam beschrieben werden.

Die Bausätze weisen jeweils zumindest zwei äußere Platten 71, 81, 91 auf, die zum Befestigen an Rücken-Wirbelkörpern vorgesehen sind, und weisen jeweils mehrere mittlere oder innere Platten 72, 82, 92 auf, die zwischen den äußeren Platten 71, 81, 91 eingesetzt werden können, um die Höhe des Käfigs einzustellen. Die Platten 71, 72, 81, 82, 91, 92 bestehen aus einem elastischen biokompatiblen Kunststoff oder aus Edelstahl, Titan, einer Titanlegierung, Tantal, einer Tantallegierung können aber auch aus Kompositen dieser Materialien gefertigt sein. Die Platten 71, 72, 81, 82, 91, 92 werden mit einem CAM-Verfahren (CAM - Computer-Aided Manufacturing, Deutsch: rechner-unterstützte Fertigung) beziehungsweise einem 3D-Druckverfahren hergestellt, beispielsweise mit selektivem Laserschmelzen SLM (Selective Laser Melting). Andere Rapid-Prototyping-Verfahren beziehungsweise computergestützte generative Herstellungsverfahren können auch zur Herstellung der Platten 71, 72, 81, 82, 91, 92 verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, Stereolithografie (STL oder SLA) von Kunststoffen, Schleif- oder Mehr-Achs-Fräsverfahren oder Digital Light Processing (DLP) von photopolymerisierbaren flüssigen Kunststoffen.

Die Platten 71, 72, 81, 82, 91, 92 weisen jeweils ein plattenförmiges Flächengebilde 73, 83, 93 auf, das die gesamten Platten 71, 72, 81, 82, 91, 92 trägt und in sich verbindet. Das Flächengebilde 73, 83, 93 kann in begrenztem Umfang flexibel und elastisch verformbar sein, so dass auch andere Flächen als Ebenen mit dem Flächengebilde 73, 83, 93 geformt werden können, so dass die Platten 71, 72, 81, 82, 91, 92 in geringem Umfang an die Form der Wirbelkörper angepasst werden kann. Von den Flächengebilden 73, 83, 93 erstrecken sich bei jeder Platte 71, 72, 81, 82, 91, 92 eine Vielzahl von Stiften 74, 84, 94 weg, die senkrecht von der Ebene der Flächengebilde 73, 83, 93 abstehen.

In den Figuren 17 bis 22 sind jeweils zwei verschiedene Typen von Platten 71, 72, 81, 82, 91, 92 dargestellt nämlich äußere Platten 71, 81, 91, die eine flache Unterseite aufweisen und bei denen sich die Stifte 74, 84, 94 nur auf einer Seite der Flächengebilde 73, 83, 93 von dem Flächengebilde 73, 83, 93 aus erstrecken, und zweitens mittlere Platten 72, 82, 92 beziehungsweise innere Platten 72, 82, 92, bei denen sich die Stifte 74, 84, 94 von beiden Seiten des Flächengebildes 73, 83, 93 aus erstrecken. Bei einem fertig aufgebauten Käfig beziehungsweise bei zusammengefügten oder zusammengesteckten Platten 71, 72, 81, 82, 91, 92 (siehe Figuren 18, 20 und 22) sind die Platten 71, 72, 81, 82, 91, 92 sandwichartig zueinander angeordnet, wobei die äußeren Platten 71, 81, 91 die beiden Deckflächen bilden und die inneren Platten 72, 82, 92 zwischen den äußeren Platten 71, 81, 91 angeordnet sind. Die äußeren Platten 71, 81, 91 können großflächig anliegend auf den zu behandelnden Wirbelkörpern befestigt werden. Hierzu können Ösen (nicht gezeigt) in dem Flächengebilde 73, 83, 93 vorgesehen sein, so dass die äußeren Platten 71, 81, 91 auf die Wirbelkörper geschraubt werden können, oder es können auf der Seite des Flächengebildes 73, 83, 93 ohne Stifte 74, 84, 94 Spitzen (nicht gezeigt) vorgesehen sein, die in den Knochen der Wirbelkörper eingesteckt werden können. Die inneren Platten 72, 82, 92 können allerdings theoretisch ebenfalls auf den zu behandelnden Wirbelkörpern befestigt werden, wenn auch mit einer geringeren Auflagefläche, so dass theoretisch auf die äußeren Platten 71, 81, 91 verzichtet werden kann.

An den ansonsten zylindrischen Stiften 74, 84, 94 sind jeweils vier Pilze 75, 85, 95 pro Stift 74, 84, 94 als Rastelemente 75, 85, 95 vorgesehen. Die Pilze 75, 85, 95 an den Spitzen der Stifte 74, 84, 94 sind nach außen (von dem Flächengebilde 73, 83, 93 weg) abgerundet. Bei der sechsten Ausführungsform (Figuren 17 und 18) und der siebten Ausführungsform (Figuren 19 und 20) bilden die Pilze 75, 85 an den Spitzen der Stifte 74, 84 Kugelabschnitte, während bei der elften Ausführungsform die Pilze 95 an den Spitzen der Stifte 94 etwas spitzer gestaltet sind. Es sind aber auch andere Abrundungen, wie beispielsweise Ellipsenabschnitte möglich. Die Pilze 75, 85, 95 die unterhalb der Pilze 75, 85, 95 an der Spitze der Stifte 74, 84, 94 angeordnet sind, die also auf den Stiften 74, 84, 94 zwischen dem Flächengebilde 73, 83, 93 und den Pilzen 75, 85, 95 die an der von dem Flächengebilde 73, 83, 93 abgewandten Seite der Stifte 74, 84, 94 angeordnet sind, haben die Form einer Kegelstumpfs beziehungsweise sind stumpf-kegelförmig. An der zum Flächengebilde 73, 83, 93 hin ausgerichteten Seite bilden die Pilze 75, 85, 95 eine ebene Greiffläche 79, 89, 99, die zum Verhaken oder Rasten mit anderen Pilzen 75, 85, 95 eingreifender Platten oder mit Ausnehmungen 76, 86, 96 eingreifender Platten geeignet sind. Die Stifte 94 und Pilze 95 der achten Ausführungsform (Figuren 21 und 22) weisen einen etwas geringeren Durchmesser auf als die der sechsten und siebten Ausführungsform, wobei die Greifflächen 99 der achten Ausführungsform etwas tiefer beziehungsweise großflächiger ausgestaltet sind als die Greifflächen 79, 89 der sechsten und siebten Ausführungsform.

In den Stiften 74, 84, 94, beziehungsweise zwischen den Pilzen 75, 85, 95 sind Nuten 76, 86, 96 als Gegenrastmittel 76, 86, 96 vorgesehen, in die die Pilze 75, 85, 95 benachbarter Platten 71, 72, 81, 82, 91, 92 eingreifen beziehungsweise verhaken oder einrasten können. Die Pilze 75, 85, 95 bilden so Rastelemente 75, 85, 95 und die Nuten 76, 86, 96 in etwa passende Gegenrastmittel 76, 86, 96. Ein weiteres Einschieben der Platten 71, 72, 81, 82, 91, 92 ist bei der der sechsten, siebten und achten Ausführungsform möglich, indem die Stifte 74, 84, 94 mit den Pilzen 75, 85, 95 in die beziehungsweise durch die Ausnehmungen 73, 83, 93 gedrückt werden.

Die Figuren 18, 20 und 22 zeigen hierzu eine schematische Querschnittansicht auf über die Pilze 75, 85, 95 miteinander verhakter Platten 71, 72, 81, 82, 91, 92 der erfindungsgemäßen Bausätze. In den Flächengebilden 73, 83, 93 der Platten 71, 72, 81, 82, 91, 92 sind zwischen den Stiften 74, 84, 94 eine Vielzahl von durchgehenden Poren 77, 87, 97 angeordnet. Die Poren 77, 87, 97 bewirken eine offene Porosität des Käfigs an der Auflagefläche zum Wirbelkörper in einer Richtung senkrecht zu den Flächengebilden 73, 83, 93 bewirken. Hierdurch kann der Knochen der Wirbelkörper leichter mit den äußeren Platten 71, 81, 91 des Käfigs verwachsen.

Die Stifte 74, 84, 94 sind zwischen den Pilzen 75, 85, 95 und den Flächengebilden 73, 83, 93 am dünnsten, so dass die Stifte 74, 84, 94 im Bereich der Verbindung zu den Flächengebilden 73, 83, 93 an leichtesten umgebogen werden können, beziehungsweise dort am beweglichsten sind, um den Rastvorgang oder das Verhaken der Pilze 75, 85, 95 mit den Nuten 76, 86, 96 zwischen den Pilzen 75, 85, 95 zu ermöglichen. Um einen erfindungsgemäßen Käfig mit Hilfe eines erfindungsgemäßen Bausatzes aufzubauen, liegen die Platten 71, 72, 81, 82, 91, 92 bevorzugt aneinander an, liegen aber nicht miteinander verhakt oder gerastet vor, so dass also die Pilze 75, 85, 95 der Stifte 74, 84, 94 benachbarter Platten 71, 72, 81, 82, 91, 92 noch nicht ineinander greifen. Zudem können die Platten 71, 72, 81, 82, 91, 92 mit einer Flüssigkeit benetzt vorliegen. In der Flüssigkeit ist bevorzugt zumindest eine pharmazeutisch wirksame Substanz enthalten, die zur Bekämpfung einer Infektion oder zum Anregen des Knochenwachstums geeignet ist. Alternativ oder zusätzlich können die Platten 71, 72, 81, 82, 91, 92 mit einer solchen pharmazeutisch wirksamen Substanz beschichtet sein.

Der Käfig kann geformt werden, indem die Platten 71, 72, 81, 82, 91, 92 über ihre Flächen ineinander gedrückt werden. Dadurch rasten die Platten 71, 72, 81, 82, 91, 92 miteinander und der Käfig wird in der gewünschten Form verfestigt. Zuvor oder während dessen können die Platten 71, 72, 81, 82, 91, 92 auch durch eine leichte elastische Verformung der Flächengebilde 73, 83, 93 verformt und an die Behandlungssituation angepasst werden. Nach dem Rasten mit zumindest einer weiteren (meist dann ebenfalls verformten) inneren Platte 72, 82, 92 stabilisieren sich die beiden so miteinander verbundenen Platten 71, 72, 81, 82, 91, 92 gegenseitig, so dass die gewählte Form verfestigt ist.

Die Platten 71, 72, 81, 82, 91, 92 können dabei mit einander rasten, indem die Pilze 75, 85, 95 die Stifte 74, 84, 94 verbundener Platten 71, 72, 81, 82, 91, 92 elastisch verformen und durch die elastische Rückstellkraft der Stifte 74, 84, 94 die Pilze 75, 85, 95 beziehungsweise die Ränder der Pilze 75, 85, 95 in die Nuten 76, 86, 96 drücken und dadurch die Bewegung benachbarter Platten 71, 72, 81, 82, 91, 92 von dem Flächengebilde 73, 83, 93 weg begrenzen (siehe Figuren 18, 20 und 22).

Die Platten 71, 72, 81, 82, 91, 92 verbinden sich dabei untereinander in der Art, dass freie Zwischenräume zwischen den miteinander verbundenen Platten 71, 72, 81, 82, 91, 92 im Bereich der Stifte 74, 84, 94, der Pilze 75, 85, 95 und der Nuten 76, 86, 96 verbleiben, so dass der aus den Platten 71, 72, 81, 82, 91, 92 geformte Käfige in den Richtungen parallel zur Ebene der Platten 71, 72, 81, 82, 91, 92 offenporig ist. Die Platten 71, 72, 81, 82, 91, 92 haben einen Querschnitt beziehungsweise eine Dicke zwischen 0,25 mm und 1,5 mm. Dieser Querschnitt reicht aus, damit sich in den Poren 77, 87, 97 und zwischen den Platten 71, 72, 81, 82, 91, 92 Knochenmaterial ausbilden beziehungsweise einwachsen kann. Der Käfig mit seinen offenen Poren 77, 87, 97 kann also als osteokonduktiv bezeichnet werden. Der aus den Platten 71, 72, 81, 82, 91, 92 geformte Käfig ist daher zum Verbinden mit dem Knochen der Wirbelkörper gut geeignet.

Der fertig aufgebaute Käfig weist zwei offene axiale Hohlräume (nicht gezeigt) auf, die durch übereinander platzieren der Platten 71, 72, 81, 82, 91, 92 entstehen, in denen hierzu passende Ausnehmungen vorhanden sind, die jedoch in den Figuren 17 bis 22 nicht zu sehen sind. Die axialen Hohlräume und die Ausnehmungen sind analog den vorangegangenen Ausführungsbeispielen aufgebaut. Die offenen axialen Hohlräume und damit die Ausnehmungen dienen daher ebenfalls dazu, dass Knochen der Wirbelkörper sie durchwachsen kann. Dazu sind die Oberflächen der offenen axialen Hohlräume und der Ausnehmungen mit autologem Knochenersatzmaterial gefüllt und, wenn gewünscht, zusätzlich mit einer das Knochenwachstum fördernden Substanz beschichtet. Der freie Querschnitt der Ausnehmungen und der offenen axialen Hohlräume beträgt etwa12 mm aber zumindest 5 mm, damit der Knochen der Wirbelkörper gut einwachsen kann. Die offenen axialen Hohlräume bilden somit das Innere des Käfigs.

Damit die offenen axialen Hohlräume gleichmäßig sind und die äußere Form des Käfigs eben ist, müssen die Platten 71, 72, 81, 82, 91, 92 fluchtend beziehungsweise passend aufeinander gerastet werden. Um dies zu erleichtern, könnten analog dem ersten Ausführungsbeispiel Positionierstifte (nicht gezeigt) als Positionierhilfen vorgesehen sein. Die Platten 71, 72, 81, 82, 91, 92 haben alle bezogen auf die Ebene der Flächengebilde 73, 83, 93 die gleiche Form, so dass sie passend aufeinander gelegt werden können.

Die Bausätze aller Ausführungsbeispiele können auch mehr als die gezeigten Platten umfassen und auch Plattengruppen mit verschiedenen Geometrien umfassen, um die Bausätze möglichst variabel zu gestalten und um sie für die Behandlung unterschiedlicher anatomischer Gegebenheiten verwendbar zu machen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

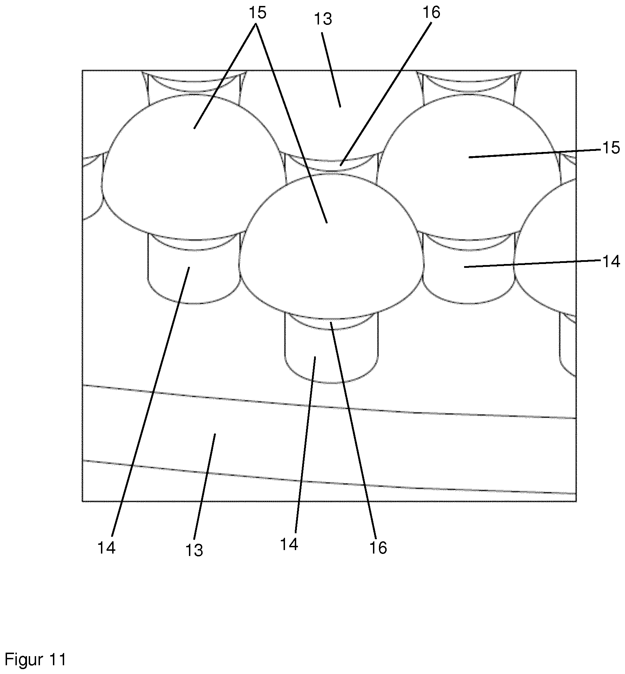

- 1, 11, 21, 71, 81, 91: Äußere Platte
- 2, 12, 22, 72, 82, 92: Mittlere Platte / Innere Platte
- 3, 13, 53, 63, 73, 83, 93: Flächengebilde
- 4, 14, 54, 64, 74, 84, 94: Stift
- 5, 15, 65, 75, 85, 95: Pilz / Rastelement
- 6, 16, 66, 76, 86, 96: Nut / Gegenrastmittel
- 7, 67, 77, 87, 97: Pore
- 8, 18: Offener axialer Hohlraum
- 9, 19: Ausnehmung
- 10: Positionierstift
- 17: Rücken-Wirbelkörper
- 27: Rand
- 55, 68: Haken / Rastelement
- 69, 79, 89, 99: Greiffläche
- A: Teilbereich aus Figur 10 vergrößert dargestellt in Figur 11

## Patentansprüche

1. Modularer Bausatz zur Herstellung eines Käfigs für die Spondylodese, der Bausatz aufweisend zumindest zwei Platten (1, 2, 11, 12, 21, 22, 61, 62, 71, 72, 81, 82, 91, 92), wobei die Platten (1, 2, 11, 12, 21, 22, 61, 62, 71, 72, 81, 82, 91, 92) aus einem biokompatiblen Material bestehen und jeweils ein Flächengebilde (3, 13, 53, 63, 73, 83, 93) aufweisen und eine Mehrzahl von sich aus dem Flächengebilde (3, 13, 53, 63, 73, 83, 93) der Platten (1, 2, 11, 12, 21, 22, 61, 62, 71, 72, 81, 82, 91, 92) heraus erstreckende Stifte (4, 14, 54, 64, 74, 84, 94) aufweisen, wobei die Stifte (4, 14, 54, 64, 74, 84, 94) jeweils mindestens ein Rastelement (5, 15, 55, 65, 68, 75, 76, 85, 86, 95, 96) aufweisen, wobei die Stifte (4, 14, 54, 64, 74, 84, 94) elastisch verformbar sind und auf dem Flächengebilde (3, 13, 53, 63, 73, 83, 93) derart dicht nebeneinander angeordnet sind, so dass durch Aufeinanderpressen von mit Stiften (4, 14, 54, 64, 74, 84, 94) besetzten Flächengebilden (3, 13, 53, 63, 73, 83, 93) mehrerer Platten (1, 2, 11, 12, 21, 22, 61, 62, 71, 72, 81, 82, 91, 92) die Rastelemente (5, 15, 55, 65, 68, 75, 76, 85, 86, 95, 96) verschiedener Platten (1, 2, 11, 12, 21, 22, 61, 62, 71, 72, 81, 82, 91, 92) miteinander rasten, wobei zumindest zwei der zumindest zwei Platten (1, 2, 11, 12, 21, 22, 61, 62, 71, 72, 81, 82, 91, 92) eine Ausnehmung (9, 19) mit einem Durchmesser von wenigstens 5 mm aufweisen.

2. Bausatz nach Anspruch 1, **dadurch gekennzeichnet, dass**
die miteinander gerasteten Platten (1, 2, 11, 12, 21, 22, 61, 62, 71, 72, 81, 82, 91, 92) einen Käfig aus miteinander gerasteten Platten (1, 2, 11, 12, 21, 22, 61, 62, 71, 72, 81, 82, 91, 92) mit zumindest einem offenen axialen Hohlraum (8, 18) bilden, wobei der Hohlraum (8, 18) einen Durchmesser von wenigstens 5 mm hat, wobei bevorzugt die miteinander gerasteten Platten (1, 2, 11, 12, 21, 22, 61, 62, 71, 72, 81, 82, 91, 92) einen Käfig aus miteinander gerasteten Platten (1, 2, 11, 12, 21, 22, 61, 62, 71, 72, 81, 82, 91, 92) mit zwei offenen axialen Hohlräumen (8, 18) bilden, wobei die beiden Hohlräume (8, 18) einen Durchmesser von jeweils wenigstens 5 mm hat.

3. Bausatz nach Anspruch 1 oder 2, **dadurch gekennzeichnet, dass**
jede der zumindest zwei Platten (1, 2, 11, 12, 21, 22, 61, 62, 71, 72, 81, 82, 91, 92) zumindest eine Ausnehmung (9, 19) mit einem Durchmesser von wenigstens 5 mm aufweist, wobei bevorzugt mit den Ausnehmungen (9, 19) der zumindest zwei Platten (1, 2, 11, 12, 21, 22, 61, 62, 71, 72, 81, 82, 91, 92) der zumindest eine offene axiale Hohlraum (8, 18) des aus den Platten (1, 2, 11, 12, 21, 22, 61, 62, 71, 72, 81, 82, 91, 92) gefertigten Käfigs nach Anspruch 2 herzustellen ist.

4. Bausatz nach einem der vorangehenden Ansprüche, **dadurch gekennzeichnet, dass**
der modulare Bausatz zur Herstellung eines Käfigs für die Spondylodese eine einstellbare Höhe aufweist und hierzu vorzugsweise zumindest drei Platten (1, 2, 11, 12, 21, 22, 61, 62, 71, 72, 81, 82, 91, 92) aufweist, damit durch die optionale Verwendung einer inneren Platte (2, 12, 22, 62, 72, 82, 92) oder von mehreren inneren Platten (2, 12, 22, 62, 72, 82, 92) verschiedene Höhen einstellbar sind.

5. Bausatz nach einem der vorangehenden Ansprüche, **dadurch gekennzeichnet, dass**
die zumindest zwei Platten (1, 2, 11, 12, 21, 22, 61, 62, 71, 72, 81, 82, 91, 92) einen abschließenden umlaufenden Rand (27) aufweisen, so dass die miteinander gerasteten Platten (1, 2, 11, 12, 21, 22, 61, 62, 71, 72, 81, 82, 91, 92) einen Käfig mit einer nach außen abgeschlossenen Wandung bilden, wobei vorzugsweise die Ränder (27) ineinander verzahnt sind.

6. Bausatz nach einem der vorangehenden Ansprüche, **dadurch gekennzeichnet, dass**
zumindest zwei äußeren Platten (1, 11, 21, 61, 71, 81, 91) der zumindest zwei Platten (1, 2, 11, 12, 21, 22, 61, 62, 71, 72, 81, 82, 91, 92), die zum direkten Verbinden mit den Wirbelkörpern (17) vorgesehen sind, durch Poren (7, 69, 79, 89, 99) in den Flächengebilden (3, 13, 53, 63, 73, 83, 93) osteokonduktiv sind und/oder die Flächengebilde (3, 13, 53, 63, 73, 83, 93) eine Befestigungs-Oberfläche ohne Stifte (4, 14, 54, 64, 74, 84, 94) aufweisen, die zum Anlegen an die Wirbelkörper (17) vorgesehen ist, wobei bevorzugt die Befestigungs-Oberfläche Spitzen oder Noppen zum Verbinden der Platten (1, 2, 11, 12, 21, 22, 61, 62, 71, 72, 81, 82, 91, 92) mit dem Knochen der Wirbelkörper (17) aufweist.

7. Bausatz nach einem der vorangehenden Ansprüche, **dadurch gekennzeichnet, dass**
zumindest drei Platten (1, 2, 11, 12, 21, 22, 61, 62, 71, 72, 81, 82, 91, 92) vorgesehen sind und dabei zumindest eine innere Platten (2, 12, 22, 62, 72, 82, 92) und zumindest eine äußere Platten (1, 11, 21, 61, 71, 81, 91), wobei immer eine innere Platte (2, 12, 22, 62, 72, 82, 92) und dazu jeweils wenigstens eine äußere Platte (1, 11, 21, 61, 71, 81, 91) eine Flächengruppe bilden, wobei jede äußere Platte (1, 11, 21, 61, 71, 81, 91) eine Ausnehmung (9, 19) aufweist, so dass diese äußere Platte (1, 11, 21, 61, 71, 81, 91) die innere Platte (2, 12, 22, 62, 72, 82, 92) der gleichen Flächengruppe oder eine andere äußere Platte (1, 11, 21, 61, 71, 81, 91) der gleichen Flächengruppe in der Ebene der Flächengebilde (3, 13, 53, 63, 73, 83, 93) formschlüssig umschließt.

8. Bausatz nach einem der vorangehenden Ansprüche, **dadurch gekennzeichnet, dass**
die Rastelemente (5, 15, 55, 65, 68, 75, 76, 85, 86, 95, 96) Pilze (5, 15, 65, 75, 85, 95), Haken (55, 68), Hinterschnitte, Rastungen und/oder Gegenrastungen (6, 16, 66, 76, 86, 96) sind.

9. Bausatz nach einem der vorangehenden Ansprüche, **dadurch gekennzeichnet, dass**
zumindest eines der zumindest einen Rastelemente (5, 15, 55, 65, 68, 75, 76, 85, 86, 95, 96) pro Stift (4, 14, 54, 64, 74, 84, 94) stumpf-kegelförmig ausgebildet ist, wobei die Längsachsen der Stifte (4, 14, 54, 64, 74, 84, 94) die Längsachsen der Kegel bilden und wobei der Mantel der Kegel nach der zu dem Flächengebilde (3, 13, 53, 63, 73, 83, 93) der zumindest einen Platte (1, 2, 11, 12, 21, 22, 61, 62, 71, 72, 81, 82, 91, 92) abgewandten Außenseite gerichtet ist.

10. Bausatz nach einem der vorangehenden Ansprüche, **dadurch gekennzeichnet, dass**
die Stifte (4, 14, 54, 64, 74, 84, 94) zwischen dem Flächengebilde (3, 13, 53, 63, 73, 83, 93) der zumindest zwei Platten (1, 2, 11, 12, 21, 22, 61, 62, 71, 72, 81, 82, 91, 92) und zumindest einem der zumindest einen Rastelemente (5, 15, 55, 65, 68, 75, 76, 85, 86, 95, 96) eine umlaufende Nut (6, 16, 66, 76, 86, 96) als Gegenrastmittel (6, 16, 66, 76, 86, 96) enthalten, in welche Rastelemente (5, 15, 55, 65, 68, 75, 76, 85, 86, 95, 96) anderer Platten (1, 2, 11, 12, 21, 22, 61, 62, 71, 72, 81, 82, 91, 92) einrasten können, bevorzugt derart einrasten, dass keine weitere Bewegung der Rastelemente (5, 15, 55, 65, 68, 75, 76, 85, 86, 95, 96) entlang der Stifte (4, 14, 54, 64, 74, 84, 94) möglich ist.

11. Bausatz nach einem der vorangehenden Ansprüche, **dadurch gekennzeichnet, dass**
benachbarte Stifte (4, 14, 54, 64, 74, 84, 94), die auf der gleichen Seite einer ersten Platte (1, 2, 11, 12, 21, 22, 61, 62, 71, 72, 81, 82, 91, 92) der zumindest zwei Platten (1, 2, 11, 12, 21, 22, 61, 62, 71, 72, 81, 82, 91, 92) angeordnet sind, einen derartigen Abstand zueinander aufweisen, dass nach einer elastischer Deformation aufgrund einer Rastung mit einem Rastelement (5, 15, 55, 65, 68, 75, 76, 85, 86, 95, 96) einer zweiten Platte (1, 2, 11, 12, 21, 22, 61, 62, 71, 72, 81, 82, 91, 92) der zumindest zwei Platten (1, 2, 11, 12, 21, 22, 61, 62, 71, 72, 81, 82, 91, 92) die Stifte (4, 14, 54, 64, 74, 84, 94) der ersten Platte (1, 2, 11, 12, 21, 22, 61, 62, 71, 72, 81, 82, 91, 92) mindestens zwei Rastungen mit zumindest zwei weiteren Rastelementen (5, 15, 55, 65, 68, 75, 76, 85, 86, 95, 96) der zweiten Platte (1, 2, 11, 12, 21, 22, 61, 62, 71, 72, 81, 82, 91, 92) ermöglichen, bevorzugt mindestens drei Rastungen mit drei weiteren Rastelementen (5, 15, 55, 65, 68, 75, 76, 85, 86, 95, 96) der zweiten Platte (1, 2, 11, 12, 21, 22, 61, 62, 71, 72, 81, 82, 91, 92) ermöglichen.

12. Bausatz nach einem der vorangehenden Ansprüche, **dadurch gekennzeichnet, dass**
der Bausatz zumindest zwei äußere Platten (1, 2, 11, 12, 21, 22, 61, 62, 71, 72, 81, 82, 91, 92) zum Verbinden mit den Wirbelkörpern (17) und zumindest eine innere Platte (2, 12, 22, 62, 71, 72, 81, 82, 91, 92) zum Einstellen der Höhe des zu erzeugenden Käfigs aufweist, wobei jede der zumindest einen inneren Platte (2, 12, 22, 62, 71, 72, 81, 82, 91, 92) auf beiden Seiten des Flächengebildes (3, 13, 53, 63, 73, 83, 93) Stifte (4, 14, 54, 64, 74, 84, 94) mit Rastelementen (5, 15, 55, 65, 68, 75, 76, 85, 86, 95, 96) aufweist und bevorzugt die zumindest zwei äußeren Platten (1, 2, 11, 12, 21, 22, 61, 62, 71, 72, 81, 82, 91, 92) nur auf einer Seite der Flächengebilde (3, 13, 53, 63, 73, 83, 93) Stifte (4, 14, 54, 64, 74, 84, 94) mit Rastelementen (5, 15, 55, 65, 68, 75, 76, 85, 86, 95, 96) aufweisen.

13. Verfahren zum Herstellen eines Käfigs für die Spondylodese mit einem Bausatz nach einem der vorangehenden Ansprüche, bei dem mehrere Platten (1, 2, 11, 12, 21, 22, 61, 62, 71, 72, 81, 82, 91, 92) gegeneinander gedrückt werden, wobei die Platten (1, 2, 11, 12, 21, 22, 61, 62, 71, 72, 81, 82, 91, 92) miteinander rasten und den Käfig ausbilden.

14. Verfahren nach Anspruch 13, **dadurch gekennzeichnet, dass**
zwischen zwei äußere Platten (1, 11, 21, 61, 71, 81, 91) je nach gewünschter Dicke des zu erzeugenden Käfigs keine, eine oder mehrere innere Platten (2, 12, 22, 62, 72, 82, 92) eingesetzt werden, wobei die Platten (1, 2, 11, 12, 21, 22, 61, 62, 71, 72, 81, 82, 91, 92) durch aufeinanderdrücken miteinander gerastet und dadurch fest miteinander verbunden werden.

15. Käfig für die Spondylodese aufgebaut aus zumindest zwei Platten (1, 2, 11, 12, 21, 22, 61, 62, 71, 72, 81, 82, 91, 92) eines Bausatzes nach einem der Ansprüche 1 bis 12 und/oder hergestellt mit einem Verfahren nach Anspruch 13 oder 14.

## Claims

1. A modular kit for building a cage for spondylodesis, the kit comprising at least two plates (1, 2, 11, 12, 21, 22, 61, 62, 71, 72, 81, 82, 91, 92), wherein the plates (1, 2, 11, 12, 21, 22, 61, 62, 71, 72, 81, 82, 91, 92) consist of a biocompatible material and each comprise a planar structure (3, 13, 53, 63, 73, 83, 93) and a plurality of pins (4, 14, 54, 64, 74, 84, 94) projecting from the planar structure (3, 13, 53, 63, 73, 83, 93) of the plates (1, 2, 11, 12, 21, 22, 61, 62, 71, 72, 81, 82, 91, 92), wherein the pins (4, 14, 54, 64, 74, 84, 94) each comprise at least one latching element (5, 15, 55, 65, 68, 75, 76, 85, 86, 95, 96), wherein the pins (4, 14, 54, 64, 74, 84, 94) are elastically deformable and are arranged sufficiently close to each other on the planar structure (3, 13, 53, 63, 73, 83, 93) such that pressing planar structures (3, 13, 53, 63, 73, 83, 93) studded with pins (4, 14, 54, 64, 74, 84, 94) of several plates (1, 2, 11, 12, 21, 22, 61, 62, 71, 72, 81, 82, 91, 92) onto each other causes the latching elements (5, 15, 55, 65, 68, 75, 76, 85, 86, 95, 96) of different plates (1, 2, 11, 12, 21, 22, 61, 62, 71, 72, 81, 82, 91, 92) to snap into each other, wherein at least two of the at least two plates (1, 2, 11, 12, 21, 22, 61, 62, 71, 72, 81, 82, 91, 92) comprise a recess (9, 19) with a diameter of at least 5 mm.

2. The kit according to claim 1, **characterized in that**
the plates which are snapped into each other (1, 2, 11, 12, 21, 22, 61, 62, 71, 72, 81, 82, 91, 92) form a cage of plates (1, 2, 11, 12, 21, 22, 61, 62, 71, 72, 81, 82, 91, 92) which are snapped into each other with at least one open axial hollow chamber (8, 18), wherein the hollow chamber (8, 18) has a diameter of at least 5 mm, wherein preferably the plates (1, 2, 11, 12, 21, 22, 61, 62, 71, 72, 81, 82, 91, 92) which are snapped into each other form a cage of plates (1, 2, 11, 12, 21, 22, 61, 62, 71, 72, 81, 82, 91, 92) which are snapped into each other with two open axial hollow chambers (8, 18), wherein the two hollow chambers (8, 18) each have a diameter of at least 5 mm.

3. The kit according to claim 1 or 2, **characterized in that**
each of the at least two plates (1, 2, 11, 12, 21, 22, 61, 62, 71, 72, 81, 82, 91, 92) comprises at least one recess (9, 19) with a diameter of at least 5 mm, wherein preferably the recesses (9, 19) of the at least two plates (1, 2, 11, 12, 21, 22, 61, 62, 71, 72, 81, 82, 91, 92) are to be used to create the at least one open axial hollow chamber (8, 18) of the cage built of the plates (1, 2, 11, 12, 21, 22, 61, 62, 71, 72, 81, 82, 91, 92) according to claim 2.

4. The kit according to any one of the preceding claims, **characterized in that**
the modular kit for building a cage for spondylodesis has an adjustable height and for this purpose preferably comprises at least three plates (1, 2, 11, 12, 21, 22, 61, 62, 71, 72, 81, 82, 91, 92) so that different heights can be set through the optional use of an inner plate (2, 12, 22, 62, 72, 82, 92) or of several inner plates (2, 12, 22, 62, 72, 82, 92).

5. The kit according to any one of the preceding claims, **characterized in that**
the at least two plates (1, 2, 11, 12, 21, 22, 61, 62, 71, 72, 81, 82, 91, 92) comprise a completing circumferential edge (27), so that the plates (1, 2, 11, 12, 21, 22, 61, 62, 71, 72, 81, 82, 91, 92) which are snapped into each other form a cage with a wall that is closed to the outside, wherein preferably the edges (27) are interlocked.

6. The kit according to any one of the preceding claims, **characterized in that**
at least two outer plates (1, 11, 21, 61, 71, 81, 91) of the at least two plates (1, 2, 11, 12, 21, 22, 61, 62, 71, 72, 81, 82, 91, 92), which are provided for direct connection to the vertebral bodies (17), are osteoconductive due to pores (7, 69, 79, 89, 99) in the planar structures (3, 13, 53, 63, 73, 83, 93) and/or the planar structures (3, 13, 53, 63, 73, 83, 93) comprise an attachment surface without pins (4, 14, 54, 64, 74, 84, 94), which is designed to be placed against the vertebral bodies (17), wherein preferably the attachment surface comprises peaks or naps for connecting the plates (1, 2, 11, 12, 21, 22, 61, 62, 71, 72, 81, 82, 91, 92) to the bone of the vertebral bodies (17).

7. The kit according to any one of the preceding claims, **characterized in that**
at least three plates (1, 2, 11, 12, 21, 22, 61, 62, 71, 72, 81, 82, 91, 92) are provided, including at least one inner plate (2, 12, 22, 62, 72, 82, 92) and at least one outer plate (1, 11, 21, 61, 71, 81, 91), wherein always one inner plate (2, 12, 22, 62, 72, 82, 92) and in addition at least one outer plate (1, 11, 21, 61, 71, 81, 91) form a planar group, wherein each outer plate (1, 11, 21, 61, 71, 81, 91) comprises a recess (9, 19), so that this outer plate (1, 11, 21, 61, 71, 81, 91) encloses the inner plate (2, 12, 22, 62, 72, 82, 92) of the same planar group or a different outer plate (1, 11, 21, 61, 71, 81, 91) of the same planar group in a form-fit manner on the plane of the planar structures (3, 13, 53, 63, 73, 83, 93).

8. The kit according to any one of the preceding claims, **characterized in that**
the latching elements (5, 15, 55, 65, 68, 75, 76, 85, 86, 95, 96) are mushrooms (5, 15, 65, 75, 85, 95), hooks (55, 68), undercuts, snap-in elements and/or counter snap-in elements (6, 16, 66, 76, 86, 96).

9. The kit according to any one of the preceding claims, **characterized in that**
at least one of the at least one latching elements (5, 15, 55, 65, 68, 75, 76, 85, 86, 95, 96) per pin (4, 14, 54, 64, 74, 84, 94) has a truncated cone shape, wherein the longitudinal axes of the pins (4, 14, 54, 64, 74, 84, 94) form the longitudinal axes of the cones and wherein the jacket of the cones faces towards the outer side that faces away from the planar structure (3, 13, 53, 63, 73, 83, 93) of the at least one plate (1, 2, 11, 12, 21, 22, 61, 62, 71, 72, 81, 82, 91, 92).

10. The kit according to any one of the preceding claims, **characterized in that**
the pins (4, 14, 54, 64, 74, 84, 94) between the planar structure (3, 13, 53, 63, 73, 83, 93) of the at least two plates (1, 2, 11, 12, 21, 22, 61, 62, 71, 72, 81, 82, 91, 92) and at least one of the at least one latching elements (5, 15, 55, 65, 68, 75, 76, 85, 86, 95, 96) contain a circumferential groove (6, 16, 66, 76, 86, 96) as a counter-latching means (6, 16, 66, 76, 86, 96), into which latching elements (5, 15, 55, 65, 68, 75, 76, 85, 86, 95, 96) of other plates (1, 2, 11, 12, 21, 22, 61, 62, 71, 72, 81, 82, 91, 92) can snap, preferably snapping into place in such a manner that no further movement of the latching elements (5, 15, 55, 65, 68, 75, 76, 85, 86, 95, 96) along the pins (4, 14, 54, 64, 74, 84, 94) is possible.

11. The kit according to any one of the preceding claims, **characterized in that** adjacent pins (4, 14, 54, 64, 74, 84, 94) which are arranged on the same side of a first plate (1, 2, 11, 12, 21, 22, 61, 62, 71, 72, 81, 82, 91, 92) of the at least two plates (1, 2, 11, 12, 21, 22, 61, 62, 71, 72, 81, 82, 91, 92) have such a distance between each other that, following an elastic deformation due to a snapped-in connection with a latching element (5, 15, 55, 65, 68, 75, 76, 85, 86, 95, 96) of a second plate (1, 2, 11, 12, 21, 22, 61, 62, 71, 72, 81, 82, 91, 92) of the at least two plates (1, 2, 11, 12, 21, 22, 61, 62, 71, 72, 81, 82, 91, 92), the pins (4, 14, 54, 64, 74, 84, 94) of the first plate (1, 2, 11, 12, 21, 22, 61, 62, 71, 72, 81, 82, 91, 92) enable at least two snapped-in connections with at least two further latching elements (5, 15, 55, 65, 68, 75, 76, 85, 86, 95, 96) of the second plate (1, 2, 11, 12, 21, 22, 61, 62, 71, 72, 81, 82, 91, 92), preferably at least three snapped-in connections with three further latching elements (5, 15, 55, 65, 68, 75, 76, 85, 86, 95, 96) of the second plate (1, 2, 11, 12, 21, 22, 61, 62, 71, 72, 81, 82, 91, 92).

12. The kit according to any one of the preceding claims, **characterized in that**
the kit comprises at least two outer plates (1, 2, 11, 12, 21, 22, 61, 62, 71, 72, 81, 82, 91, 92) for connection to the vertebral bodies (17) and at least one inner plate (2, 12, 22, 62, 71, 72, 81, 82, 91, 92) for setting the height of the cage to be built, wherein each of the at least one inner plate (2, 12, 22, 62, 71, 72, 81, 82, 91, 92) comprises pins (4, 14, 54, 64, 74, 84, 94) with latching elements (5, 15, 55, 65, 68, 75, 76, 85, 86, 95, 96) on both sides of the planar structure (3, 13, 53, 63, 73, 83, 93), and preferably the at least two outer plates (1, 2, 11, 12, 21, 22, 61, 62, 71, 72, 81, 82, 91, 92) comprise pins (4, 14, 54, 64, 74, 84, 94) with latching elements (5, 15, 55, 65, 68, 75, 76, 85, 86, 95, 96) on only one side of the planar structures (3, 13, 53, 63, 73, 83, 93).

13. A method for building a cage for spondylodesis with a kit according to any one of the preceding claims, in which several plates (1, 2, 11, 12, 21, 22, 61, 62, 71, 72, 81, 82, 91, 92) are pressed against each other, wherein the plates (1, 2, 11, 12, 21, 22, 61, 62, 71, 72, 81, 82, 91, 92) snap into each other, thus forming the cage.

14. The method according to claim 13, **characterized in that**
depending on the desired thickness of the cage to be built, no, one or several inner plates (2, 12, 22, 62, 72, 82, 92) is/are inserted between two outer plates (1, 11, 21, 61, 71, 81, 91), wherein the plates (1, 2, 11, 12, 21, 22, 61, 62, 71, 72, 81, 82, 91, 92) are snapped into each other by being pressed on top of each other and as a result are firmly connected to each other.

15. A cage for spondylodesis constructed of at least two plates (1, 2, 11, 12, 21, 22, 61, 62, 71, 72, 81, 82, 91, 92) from a kit according to any one of claims 1 to 12 and/or built using a method according to claim 13 or 14.

## Revendications

1. Kit modulaire pour la fabrication d'une cage pour la spondylodèse, le kit présentant au moins deux plaques (1, 2, 11, 12, 21, 22, 61, 62, 71, 72, 81, 82, 91, 92), où les plaques (1, 2, 11, 12, 21, 22, 61, 62, 71, 72, 81, 82, 91, 92) sont constituées d'un matériau biocompatible et présentent respectivement une structure plane (3, 13, 53, 63, 73, 83, 93) et une multiplicité de goupilles (4, 14, 54, 64, 74, 84, 94) pointant hors de la structure plane (3, 13, 53, 63, 73, 83, 93) des plaques (1, 2, 11, 12, 21, 22, 61, 62, 71, 72, 81, 82, 91, 92), où les goupilles (4, 14, 54, 64, 74, 84, 94) présentent respectivement au moins un élément de blocage (5, 15, 55, 65, 68, 75, 76, 85, 86, 95, 96), où les goupilles (4, 14, 54, 64, 74, 84, 94) sont déformables de manière élastique et sont disposées de manière si dense les unes à côté des autres sur la structure plane (3, 13, 53, 63, 73, 83, 93) que, par la compression les unes sur les autres des structures planes (3,13, 53, 63, 73, 83, 93) munies de goupilles (4, 14, 54, 64, 74, 84, 94), plusieurs plaques (1, 2, 11, 12, 21, 22, 61, 62, 71, 72, 81, 82, 91, 92) bloquent ensemble les éléments de blocage (5, 15, 55, 65, 68, 75, 76, 85, 86, 95, 96) de diverses plaques (1, 2, 11, 12, 21, 22, 61, 62, 71, 72, 81, 82, 91, 92), où au moins deux des au moins deux plaques (1, 2, 11, 12, 21, 22, 61, 62, 71, 72, 81, 82, 91, 92) présentent une cavité (9, 19) avec un diamètre d'au moins 5 mm.

2. Kit selon la revendication 1, **caractérisé en ce que**
les plaques (1, 2, 11, 12, 21, 22, 61, 62, 71, 72, 81, 82, 91, 92) bloquées ensemble forment une cage à base de plaques (1, 2, 11, 12, 21, 22, 61, 62, 71, 72, 81, 82, 91, 92) bloquées ensemble avec au moins un espace creux (8, 18) axial ouvert, où l'espace creux (8, 18) a un diamètre d'au moins 5 mm, où, de préférence, les plaques (1, 2, 11, 12, 21, 22, 61, 62, 71, 72, 81, 82, 91, 92) bloquées ensemble forment une cage à base de plaques (1, 2, 11, 12, 21, 22, 61, 62, 71, 72, 81, 82, 91, 92) bloquées ensemble avec deux espaces creux (8, 18) axiaux ouverts, où les deux espaces creux (8, 18) ont respectivement un diamètre d'au moins 5 mm.

3. Kit selon la revendication 1 ou la revendication 2, **caractérisé en ce que**
chacune des au moins deux plaques (1, 2, 11, 12, 21, 22, 61, 62, 71, 72, 81, 82, 91, 92) présente au moins une cavité (9, 19) avec un diamètre d'au moins 5 mm, où, de préférence, avec les cavités (9, 19) des au moins deux plaques (1, 2, 11, 12, 21, 22, 61, 62, 71, 72, 81, 82, 91, 92), l'au moins un espace creux (8, 18) de la cage fabriquée à partir des plaques (1, 2, 11, 12, 21, 22, 61, 62, 71, 72, 81, 82, 91, 92) peut être fabriqué selon la revendication 2.

4. Kit selon l'une des revendications précédentes, **caractérisé en ce que**
le kit modulaire pour la fabrication d'une cage pour la spondylodèse présente une hauteur réglable et présente à cet effet de préférence au moins trois plaques (1, 2, 11, 12, 21, 22, 61, 62, 71, 72, 81, 82, 91, 92), afin qu'ainsi, par l'utilisation optionnelle d'une plaque (2, 12, 22, 62, 72, 82, 92) intérieure ou de plusieurs plaques (2, 12, 22, 62, 72, 82, 92) intérieures, diverses hauteurs sont réglables.

5. Kit selon l'une des revendications précédentes, **caractérisé en ce que**
les au moins deux plaques (1, 2, 11, 12, 21, 22, 61, 62, 71, 72, 81, 82, 91, 92) présentent un bord (27) périphérique terminal de sorte que les plaques (1, 2, 11, 12, 21, 22, 61, 62, 71, 72, 81, 82, 91, 92) bloquées ensemble forment une cage avec une paroi fermée vers l'extérieur, où, de préférence, les bords (27) sont indentés les uns dans les autres.

6. Kit selon l'une des revendications précédentes, **caractérisé en ce qu'**au
moins deux plaques (1, 11, 21, 61, 71, 81, 91) extérieures parmi les au moins deux plaques (1, 2, 11, 12, 21, 22, 61, 62, 71, 72, 81, 82, 91, 92) qui sont prévues pour une liaison directe avec les corps vertébraux (17), sont ostéo-conductifs dans les structure planes (3, 13, 53, 63, 73, 83, 93) par des pores (7, 69, 79, 89, 99) et/ou les structure planes (3, 13, 53, 63, 73, 83, 93) présentent une surface de fixation sans goupilles (4, 14, 54, 64, 74, 84, 94) qui est prévue pour une application sur les corps vertébraux (17), où, de préférence, la surface de fixation présente des pointes ou des boucles pour la liaison des plaques (1, 2, 11, 12, 21, 22, 61, 62, 71, 72, 81, 82, 91, 92) avec l'os des corps vertébraux (17).

7. Kit selon l'une des revendications précédentes, **caractérisé en ce qu'**au
moins trois plaques (1, 2, 11, 12, 21, 22, 61, 62, 71, 72, 81, 82, 91, 92) sont prévues et dans ce cas, au moins une plaque (2, 12, 22, 62, 72, 82, 92) intérieure et au moins une plaque (1, 11, 21, 61, 71, 81, 91) extérieure sont prévues, où toujours une plaque (2, 12, 22, 62, 72, 82, 92) intérieure et avec elle respectivement au moins une plaque (1, 11, 21, 61, 71, 81, 91) extérieure forment une structure plane, où chaque plaque (1, 11, 21, 61, 71, 81, 91) extérieure présente une cavité (9, 19), de sorte que cette plaque (1, 11, 21, 61, 71, 81, 91) extérieure entoure par complémentarité des formes la plaque (2, 12, 22, 62, 72, 82, 92) intérieure de la même structure plane ou une autre plaque (1, 11, 21, 61, 71, 81, 91) extérieure de la même structure plane dans le plan des structure planes (3, 13, 53, 63, 73, 83, 93).

8. Kit selon l'une des revendications précédentes, **caractérisé en ce que**
les éléments de blocage (5, 15, 55, 65, 68, 75, 76, 85, 86, 95, 96) sont des butées en champignon (5, 15, 65, 75, 85, 95), des crochets (55, 68), des contre-dépouilles, des butées et/ou des butées complémentaires (6, 16, 66, 76, 86, 96).

9. Kit selon l'une des revendications précédentes, **caractérisé en ce qu'**au
moins un parmi les au moins un élément de blocage (5, 15, 55, 65, 68, 75, 76, 85, 86, 95, 96) est conçu tronconique par goupille (4, 14, 54, 64, 74, 84, 94), où les axes longitudinaux des goupilles (4, 14, 54, 64, 74, 84, 94) forment les axes longitudinaux des cônes et où l'enveloppe des cônes est orientée vers le côté extérieur opposé à la structure plane (3, 13, 53, 63, 73, 83, 93) de l'au moins une plaque (1, 2, 11, 12, 21, 22, 61, 62, 71, 72, 81, 82, 91, 92).

10. Kit selon l'une des revendications précédentes, **caractérisé en ce que**
les goupilles (4, 14, 54, 64, 74, 84, 94) entre la structure plane (3, 13, 53, 63, 73, 83, 93) des au moins deux plaques (1, 2, 11, 12, 21, 22, 61, 62, 71, 72, 81, 82, 91, 92) et au moins un des au moins un élément de blocage (5, 15, 55, 65, 68, 75, 76, 85, 86, 95, 96) contiennent une rainure (6, 16, 66, 76, 86, 96) circonférentielle servant de butée complémentaire (6, 16, 66, 76, 86, 96), dans laquelle des éléments de blocage (5, 15, 55, 65, 68, 75, 76, 85, 86, 95, 96) d'autres plaques (1, 2, 11, 12, 21, 22, 61, 62, 71, 72, 81, 82, 91, 92) peuvent se bloquer, de préférence, se bloquer de telle manière qu'aucun autre déplacement des éléments de blocage (5, 15, 55, 65, 68, 75, 76, 85, 86, 95, 96) n'est possible le long des goupilles (4, 14, 54, 64, 74, 84, 94).

11. Kit selon l'une des revendications précédentes, **caractérisé en ce que**
des goupilles (4, 14, 54, 64, 74, 84, 94) voisines, qui sont disposées sur le même côté d'une première plaque (1, 2, 11, 12, 21, 22, 61, 62, 71, 72, 81, 82, 91, 92) parmi les au moins deux plaques (1, 2, 11, 12, 21, 22, 61, 62, 71, 72, 81, 82, 91, 92) présentent entre elles un espace tel, qu'après une déformation élastique en raison d'un blocage avec un élément de blocage (5, 15, 55, 65, 68, 75, 76, 85, 86, 95, 96) d'une deuxième plaque (1, 2, 11, 12, 21, 22, 61, 62, 71, 72, 81, 82, 91, 92) parmi les au moins deux plaques (1, 2, 11, 12, 21, 22, 61, 62, 71, 72, 81, 82, 91, 92), les goupilles (4,14, 54, 64, 74, 84, 94) de la première plaque (1, 2, 11, 12, 21, 22, 61, 62, 71, 72, 81, 82, 91, 92) permettent au moins deux blocages avec au moins deux autres éléments de blocage (5, 15, 55, 65, 68, 75, 76, 85, 86, 95, 96) de la deuxième plaque (1, 2, 11, 12, 21, 22, 61, 62, 71, 72, 81, 82, 91, 92), de préférence, permettent au moins trois blocages avec trois autres éléments de blocage (5, 15, 55, 65, 68, 75, 76, 85, 86, 95, 96) de la deuxième plaque (1, 2, 11, 12, 21, 22, 61, 62, 71, 72, 81, 82, 91, 92).

12. Kit selon l'une des revendications précédentes, **caractérisé en ce que**
le kit présente au moins deux plaques (1, 2, 11, 12, 21, 22, 61, 62, 71, 72, 81, 82, 91, 92) extérieures pour la liaison avec les corps vertébraux (17) et au moins une plaque (2, 12, 22, 62, 71, 72, 81, 82, 91, 92) intérieure pour le réglage de la hauteur de la cage à créer, où chacune des au moins une plaque (2, 12, 22, 62, 71, 72, 81, 82, 91, 92) intérieure présente sur les deux côtés de la structure plane (3, 13, 53, 63, 73, 83, 93) des goupilles (4, 14, 54, 74, 84, 94) avec des éléments de blocage (5, 15, 55, 65, 68, 75, 76, 85, 86, 95, 96), et de préférence les au moins deux plaques (1, 2, 11, 12, 21, 22, 61, 62, 71, 72, 81, 82, 91, 92) extérieures ne présentent des goupilles (4, 14, 54, 64, 74, 84, 94) avec des éléments de blocage (5, 15, 55, 65, 68, 75, 76, 85, 86, 95, 96) que sur un côté des structure planes (3, 13, 53, 63, 73, 83, 93).

13. Procédé de fabrication d'une cage pour la spondylodèse avec un kit selon l'une des revendications précédentes, chez lequel plusieurs plaques (1, 2, 11, 12, 21, 22, 61, 62, 71, 72, 81, 82, 91, 92) sont pressées les unes contre les autres, où les plaques (1, 2, 11, 12, 21, 22, 61, 62, 71, 72, 81, 82, 91, 92) se bloquent ensemble et forment une cage.

14. Procédé selon la revendication 13, **caractérisé en ce qu'**entre
deux plaques (1, 11, 21, 61, 71, 81, 91) extérieures, selon l'épaisseur désirée de la cage à fabriquer, on insère aucune, une ou plusieurs plaques (2, 12, 22, 62, 72, 82, 92) intérieures, où les plaques (1, 2, 11, 12, 21, 22, 61, 62, 71, 72, 81, 82, 91, 92) sont verrouillées ensemble par une compression des unes sur les autres et qu'elles sont ainsi solidement reliées entre elles.

15. Cage pour la spondylodèse fabriquée à partir d'au moins deux plaques (1, 2, 11, 12, 21, 22, 61, 62, 71, 72, 81, 82, 91, 92) d'un kit selon l'une des revendications 1 à 12 et/ou fabriquée avec un procédé selon la revendication 13 ou la revendication 14.
